# EUROPÄISCHE PATENTSCHRIFT

(11) **EP 2 047 480 B1**
(45) Veröffentlichungstag und Bekanntmachung des Hinweises auf die Patenterteilung: **04.11.2009**
(21) Anmeldenummer: 06762863.6
(22) Anmeldetag: 27.07.2006
(51) Int. Cl.: G21K 1/04

(54) **BESTRAHLUNGSVORRICHTUNG UND KOLLIMATOR**
IRRADIATION DEVICE AND COLLIMATOR
DISPOSITIF D'IRRADIATION ET COLLIMATEUR

(43) Veröffentlichungstag der Anmeldung: 15.04.2009
(73) Patentinhaber: Deutsches Krebsforschungszentrum Stiftung des öffentlichen Rechts, 69120 Heidelberg (DE)
(72) Erfinder: ECHNER, Gernot, 69257 Wiesenbach (DE)
(74) Vertreter: Weber, Walter
(86) Internationale Anmeldenummer: PCT/EP2006/007471
(87) Internationale Veröffentlichungsnummer: WO 2008/011900

(56) Entgegenhaltungen:
- DE-A1- 1 589 432
- FR-A- 2 088 186

## Beschreibung

Die Erfindung betrifft eine Bestrahlungsvorrichtung mit einem Kollimator zum Begrenzen energiereicher Strahlen, die von einer im wesentlichen punktförmigen Strahlungsquelle ausgehend auf ein Behandlungsobjekt gerichtet sind und der Strahlenbehandlung, insbesondere der stereotaktischen Konformationsbestrahlung von Tumoren, dient, wobei der Kollimator eine als Irisblende mit Stellmitteln ausgebildete Strahlbegrenzung zur Erzielung einer variablen Blendenöffnung aufweist, wobei eine Einrichtung vorgesehen ist, durch welche die durch den Kollimator begrenzten Strahlen allseitig auf das Behandlungsobjekt richtbar sind, und wobei mittels einer Steuerung die Parameter Bestrahlungsrichtung, Bestrahlungsfläche, Bestrahlungsintensität und -zeit derart steuerbar sind, daß ein räumliches Dosierungsprofil der Strahlenapplikation erzielbar ist.

Die Erfindung betrifft weiterhin einen Kollimator zum Begrenzen energiereicher Strahlen, die von einer im wesentlichen punktförmigen Strahlungsquelle ausgehend auf ein Behandlungsobjekt gerichtet sind und der Strahlenbehandlung insbesondere der stereotaktischen Konformationsbestrahlung von Tumoren dient, wobei der Kollimator eine als Irisblende mit Stellmitteln ausgebildete Strahlbegrenzung zur Erzielung einer variablen Blendenöffnung aufweist.

Bestrahlungsvorrichtungen mit Kollimatoren zum Begrenzen eines Bündels energiereicher Strahlen werden zur Behandlung, insbesondere von Tumoren, eingesetzt. Ebenso arbeiten bilderzeugende Vorrichtungen, wie Röntgenstrahlen, mit Kollimatoren. Dabei dienen die Kollimatoren zur Begrenzung der Strahlen, damit neben dem Diagnose- bzw. Behandlungsbereich liegendes gesundes Gewebe möglichst gut vor Strahlen geschützt wird, um Schäden zu vermeiden oder auf ein Minimum zu reduzieren.

Dabei muß zwischen Diagnose- und Therapiestrahlen unterschieden werden. Die ersteren müssen so energiearm sein, daß sie möglichst keine Schäden an einem Gewebe verursachen können. Sie dienen nur einer Bilderzeugung, damit für den Arzt beispielsweise die Lage eines Tumors und des umliegenden, möglicherweise für eine Bestrahlungsbehandlung kritischen Gewebes, wie Nerven, in Vorbereitung einer Behandlung sichtbar wird. Da das umliegende Gewebe somit sichtbar sein muß, muß auch ein Bereich außerhalb eines Tumors oder sonstigen zu diagnostizierenden Bereichs den Strahlen ausgesetzt werden. Sinnvoll ist wegen der meist eher runden Form von Tumoren oder sonstiger zu diagnostizierender Bereiche eine Erfassung eines runden Abbildungsfeldes, das natürlich nicht größer sein soll als es für den Arzt von Interesse ist, da auch Röntgenstrahlen gewebeschädigend sind. Die Therapiestrahlen dagegen müssen so energiereich sein, daß man mit ihnen krankes Gewebe, wie Tumore, zerstören kann. Sie würden also auch das umliegende Gewebe zerstören. Darum muß die Abschirmung nach Möglichkeit die Strahlen in der exakten Form des kranken Gewebes begrenzen.

Bei der Applikation der energiereichen Therapiestrahlen - diesem Zweck dienen auch die erfindungsgemäße Bestrahlungsvorrichtung beziehungsweise der erfindungsgemäße Kollimator - kommt daher dem Schutz des umliegenden Gewebes eine besonders hohe Bedeutung zu, weil davon oftmals die Lebensqualität des Patienten nach der Behandlung abhängt. Derartige Nebenfolgen einer Bestrahlung durch eine Beeinträchtigung gesunden Gewebes können zwar nicht völlig ausgeschlossen werden, sie müssen jedoch auf das technisch mögliche Minimum reduziert werden. Entsprechend dieser grundlegenden Bedeutung, die bei energiereichen Strahlen im Gegensatz zu Röntgenstrahlen auch bei Kurzzeitbestrahlung sehr schwerwiegend ist, arbeitet die Fachwelt seit vielen Jahrzehnten daran, Bestrahlungsvorrichtungen für eine immer exakter werdende Strahlenapplikation zu entwickeln, um die Nebenfolgen einer Therapiebestrahlung soweit wie möglich abzusenken:

Ursprünglich waren die Bestrahlungsvorrichtungen sowie bilderzeugende Vorrichtungen mit Kollimatoren ausgestattet, die derart beschaffen waren, daß mit ihnen ein Bestrahlungsfeld lediglich der Größe, aber nicht der Form nach begrenzbar war. Dies hat bei Röntgenstrahlen zur Bilderzeugung keine schwerwiegende Beeinträchtigung des Patienten zur Folge, da diese nur bei langer Einwirkung, die für die Bilderzeugung nicht erforderlich ist, zu Schäden führt, oder dann, wenn sie bei einem Patienten innerhalb kurzer Zeit sehr oft angewendet wird. Erst die sehr energiereiche therapeutische Bestrahlung - bei der beispielsweise Tumorgewebe zerstört werden soll - führt im Überstrahlungsbereich, also außerhalb des zu bestrahlenden kranken Gewebes zu einer Schädigung von gesundem Gewebe, da die Einwirkung bezüglich Intensität und Zeit bis zur Zerstörung des kranken Gewebes andauern muß.

Solche Überstrahlungsbereiche kamen zum einen dadurch zustande, daß die Kontur des kranken Gewebes durch die Kollimatoren nicht oder unzureichend nachgebildet wurde, zum anderen dadurch, daß an den Begrenzungen des Bestrahlungsbereichs Halbschatten entstanden, da dort, insbesondere bei großen Bestrahlungsfeldern, nicht die volle Stärke des Abschirmungsmaterials zur Verfügung stand, weil es nicht parallel zu den Strahlen ausgerichtet war. Diese Halbschatten entstanden bei den energiereichen Therapiestrahlen in viel größeren Bereichen, da die Dicke der erforderlichen Abschirmung ein vielfaches dessen beträgt, was bei den verhältnismäßig energieschwachen Röntgenstrahlen erforderlich ist. Bei vielen Bestrahlungsvorrichtungen kam es auch zu Leckstrahlen, die durch Spalte zwischen aneinandergrenzenden Abschirmplatten hindurchtraten.

Ein Beispiel für einen den Strahl nur der Größe nach begrenzenden Kollimator älterer Bauart ist die US 2,675,486. Diese Schrift betrifft einen Kollimator zur Begrenzung energiereicher Strahlen, der vier Strahlbegrenzungsblöcke aufweist, welche mittels aneinandergrenzenden Seitenflächen derart in einer Ebene verschiebbar sind, daß damit eine quadratische Strahlbegrenzung unterschiedlicher Größen einstellbar ist. Da Tumore keine quadratische, sondern eher eine abgerundete Form haben, ergibt sich ein großer Überstrahlungsbereich an den Ecken. Außerdem kam es bei großen Bestrahlungsfeldern zu großen Halbschattenbereichen, da die Blockbegrenzungen nicht mehr parallel zum divergierenden Strahlengang verlaufen.

Die Fachwelt bemühte sich deshalb darum, diese Probleme zu entschärfen:

Von einem Kollimator der vorgenannten Art ausgehend schlägt die DE 20 53 089 A1 daher für den Bereich der Röntgenbilderzeugung vor, Abschirmungskörper in Form von aneinandergrenzenden Dreiecken vorzusehen, um mit dieser irisartigen Kollimatorausgestaltung ein annähernd kreisförmiges - also der Form eines Bestrahlungsbereichs eher entsprechendes - Bestrahlungsfeld zu erzielen, so daß eine durch die Ecken der vorgenannten quadratischen Strahlbegrenzung hervorgerufene, in etwa dreißigprozentige Überstrahlung vermieden wird. Die verbleibende Überstrahlung sowie eine Halbschattenbildung stellen hier jedoch kein gravierendes Problem dar, da es lediglich um Röntgenstrahlen zur Bilderzeugung, jedoch nicht um eine therapierende Bestrahlung mit den wesentlich energiereicheren Strahlen geht. Bei Röntgenstrahlen sind die Blendenblätter wesentlich dünner als bei therapierender Strahlung, so daß es zusätzlich zum vorgeschlagenen irisartigen Kollimator ausreicht, wenn fokusnahe Blendenblätter den von der Strahlungsquelle ausgehenden Strahl nur grob eingrenzen, indem afokale Strahlen in der Ebene des Anodentellers der Röntgenquelle unterbunden werden, was dazu dient, Geräteabstrahlungen in den Raum zu vermeiden. Die Eingrenzung auf ein rundes Bestrahlungsfeld reicht für die Strahlbegrenzung aus, da eine bilderzeugende Strahlung auch gar nicht so exakt eingegrenzt werden soll wie ein Therapiestrahl. Schließlich soll das angrenzende Gewebe ebenfalls abgebildet werden, damit ein Arzt die Lage des Tumors auch im Verhältnis zu diesem angrenzenden Gewebe beurteilen kann.

Die DE 15 89 432 A1 schlägt für den Einsatz der hier in Frage stehenden ionisierenden, also für die Tumorbehandlung geeigneten, energiereichen Strahlen einen Kollimator vor, bei dem aneinandergrenzende keilförmige Strahlungsabschirmungskörper in einer Ebene derart verschiebbar sind, daß sechseckige, achteckige oder rechteckige Öffnungen zusammengestellt werden können, also ein Vieleck entsprechend der Anzahl der Blendenblätter. Dieser Kollimator bildet jedoch eine Tumorform nur sehr unzureichend nach und gegen einen Halbschatten durch die nicht in Richtung des Strahlengangs ausgerichteten Vorderkanten der Blendenblätter sind keine Vorkehrungen getroffen. Auch die Leckstrahlung wird hier nur unzureichend verhindert. Die Blendenblätter werden zwar an ihren Stoßstellen von in diese eingelassenen Laschen überdeckt, die jedoch die Leckstrahlung, die durch die an den Stoßstellen entstehenden Spalte hindurchtritt, aufgrund der geringen Dicke der Laschen nur sehr unzureichend abschirmen. Bei großen Bestrahlungsfeldern, bei denen der Strahlengang sehr schräg zu der Begrenzung des Abschirmungsmaterials verläuft, tritt ein besonders großer Halbschatten auf.

Auch die DE 10 37 035 B geht von einem Kollimator der Art der erstgenannten Schrift aus und sieht für energiereiche Therapiestrahlen vor, die vier Strahlbegrenzungsblöcke entlang einer schrägen Linie in zwei Teile zu teilen, wobei die Linie zu der Stelle verläuft, an der sich die Innen- und Endfläche (also die an den nächsten Block grenzende Fläche) treffen. Dadurch erhält man von jedem Block ein Haupt- und ein Nebenteil, welche gegenseitig verschiebbar sind. Dadurch wird die Bildung verschiedener Umrisse ermöglicht, was ebenfalls die Überstrahlung gegenüber einer quadratischen Strahlbegrenzung reduziert. Allerdings ist die Nachbildung der Form eines Tumors oder eines sonstigen zu bestrahlenden Bereichs nur sehr unzureichend gelöst und das Halbschattenproblem wird nicht gelöst. Lediglich Leckstrahlen werden durch gegenseitige Schwalbenschwanzführungen im Aneinandergrenzungsbereich der Blendenblätter unterbunden.

Eine Lösung des Halbschattenproblems lehrt schließlich die DE 15 64 765 A1. Diese Schrift geht ebenfalls von einem Kollimator der Art der erstgenannten Schrift mit vier aneinandergrenzenden, in einer Ebene verschiebbaren Strahlungsbegrenzungsblöcken aus. Sie setzt sich zum Ziel, ein scharf abgegrenztes Feld, also ein Feld ohne Halbschatten zu erzielen. Es wird vorgeschlagen, zu diesem Zweck die Blöcke derart auszugestalten und schwenkbar zu lagern, daß die die Strahlungsbegrenzung bildenden Stirnflächen bei jeder Einstellung auf die Strahlenquelle gerichtet sind. Dadurch schirmt immer das Material der Blöcke in voller Stärke die Strahlung ab. Allerdings lassen sich mit diesem Kollimator wiederum nur quadratische Bestrahlungsfelder bilden, so daß hier wieder große Überstrahlungsbereiche an den Ecken in Kauf genommen werden mußten.

Sowohl das Problem der Leckstrahlen, als auch das Halbschattenproblem geht die FR 2 524 690 A an. Diese Schrift sieht zur Halbschattenverhinderung vor, aneinandergrenzende, in einer Ebene verschiebbare und gleichzeitig eine Drehbewegung vollziehende Platten in mehreren Ebenen anzuordnen, so daß eine gestufte, pyramidenstumpfförmige Strahlbegrenzungsöffnung entsteht. Auf diese Weise wird der Halbschatten im wesentlichen vermieden. Die Leckstrahlen werden dadurch vermieden, daß die Stoßstellen aneinandergrenzender Platten durch die unterschiedlichen Fensteröffnungen auf den verschiedenen Ebenen nicht mehr fluchten. Allerdings fluchten sie im geschlossenen Zustand, so daß in diesem die Strahlenquelle uneingeschaltet oder abgeschirmt sein müßte. Ein Nachteil dieses Kollimators besteht in der äußerst aufwendigen Mechanik, um die Platten aller Ebenen in entsprechender Weise zu verschieben und dabei zu drehen, damit die pyramidenstumpfförmige Strahlbegrenzungsöffnung entsteht. Ein weiterer Nachteil dieses Lösungsvorschlags besteht darin, daß als Bestrahlungsfeldbegrenzung nur Vielecke - in Abhängigkeit von der Anzahl der Platten - gebildet werden können und eine Formgebung in der wirklichen Kontur eines Tumors nicht möglich ist. Aufgrund der aufwendigen Mechanik wird eine viereckige Strahlbegrenzung bevorzugt. Diese weicht von der wirklichen Kontur eines Tumors jedoch erheblich ab.

Um Tumorformen besser nachbilden und insbesondere bei entsprechend dicken Abschirmmaterial die Überstrahlung auf ein Minimum reduzieren zu können, ging man schließlich dazu über, wechselbare feste Kollimatoren zu verwenden. Dabei wurde die Tumorform aus verschiedenen räumlichen Richtungen erfaßt und es wurden für jede Bestrahlung mehrere feste Kollimatoren hergestellt, die dann für die Bestrahlung aus den verschiedenen Richtungen eingesetzt wurden. Dabei hatte man den Vorteil exakter Formgebung und auch die Möglichkeit, die Begrenzungen exakt dem Strahlengang anzupassen, wodurch kein Halbschatten mehr auftritt. Der Nachteil bestand jedoch in dem umständlichen Verfahren mit ständigem Kollimatorwechsel, der viel Zeit an wertvollen Geräten beanspruchte sowie in dem Aufwand für die Herstellung vieler Kollimatoren für jede Bestrahlung, die danach nicht mehr zu gebrauchen waren, da sie zur Anwendung für einen Patienten bestimmt und auch für diesen nur in einem zeitlich eng begrenzten Rahmen verwendbar waren. Letzteres aufgrund des Umstandes, daß sich auch die Form des Tumors bei einem Patienten durch Wachstum, Rückbildung oder Formveränderung ständig ändert.

Um diesen Aufwand zu verringern, wurden Multileafkollimatoren geschaffen, bei denen mit einer Vielzahl von schmalen, eng aneinanderliegenden Leaves (also Blendenblättern) die Gestalt des Tumors mittels Stellbewegungen der Leaves nachgebildet werden konnte. Zunächst hatten diese Multileafkollimatoren zwar den Vorteil, daß sehr schnell fast beliebige Formen einstellbar waren, sie hatten jedoch den Nachteil einer sehr aufwendigen Mechanik mit Stellmitteln für jedes Leaf und den weiteren Nachteil, daß an jeder Begrenzung des Bestrahlungsfeldes durch ein Leaf, je nach Entfernung desselben von der Achse des Strahlengangs, ein mehr oder weniger großer Halbschatten auftrat.

Zur Vermeidung solcher Halbschatten wurde von der EP 1 153 397 B1 vorgeschlagen, die Leaves mit verstellbaren Vorderkanten auszustatten, wobei eine Mechanik sie immer parallel zum Strahlengang stellt. Dies hat allerdings eine noch aufwendigere Mechanik des Multileafkollimators zur Folge.

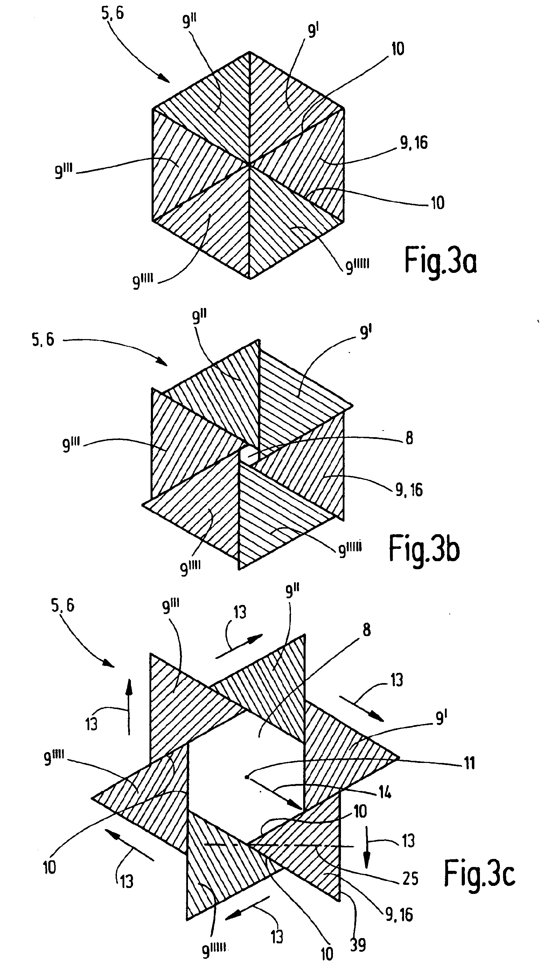

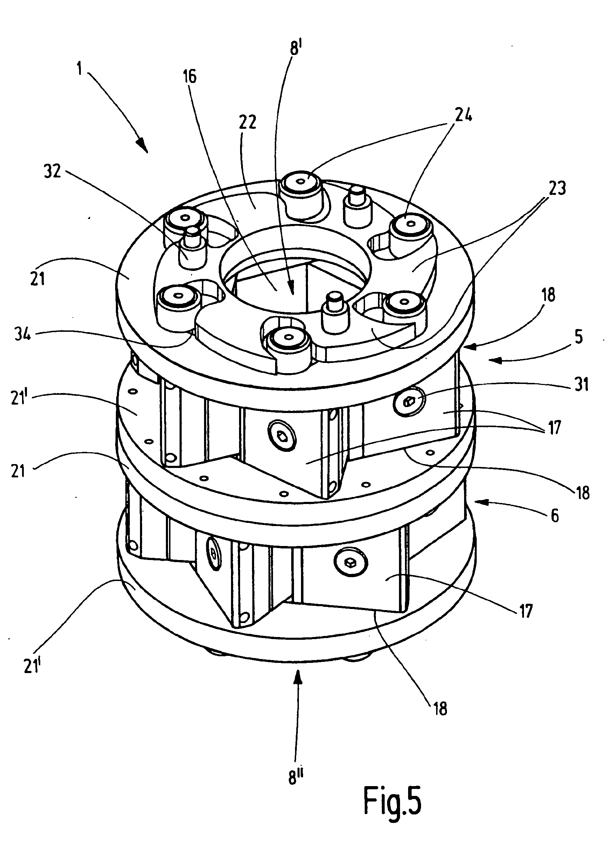

Um diese aufwendige Mechanik zu vermeiden und in der Formung einer zu bestrahlenden Fläche noch flexibler zu sein, wurde schließlich von der DE 199 22 656 A1 eine Scannvorrichtung mit einer Kollimatoröffnung vorgeschlagen, die derart klein ausgebildet ist, daß das zu bestrahlende Objekt bezüglich seiner zu bestrahlenden Bereiche mit ausreichender Genauigkeit bestrahlbar ist (Fig. 3). Beim vorgenannten Vorschlag führt eine kleine Kollimatoröffnung zwar zu einer großen Genauigkeit und verhinderte eine Halbschattenbildung, jedoch bedingte dies einen hohen Zeitaufwand für das Abscannen - bei einer großen Blendenöffnung kann schneller abgescannt werden, es ist jedoch die erforderliche Genauigkeit nicht möglich. Auch die Verwendung von Mehrlochplatten zur Erzeugung eines Bündels aus mehreren Scannstrahlen (Fig. 5 und 5a) führte dabei noch nicht zu einer befriedigenden Bestrahlungszeitverkürzung. Die Mehrlochplatte war bezüglich der Bestrahlungsfläche festgelegt und zur genauen Bestrahlung der Randbcreiche mußten noch kleinere Blendenöffnungen verwendet, also die Platten gewechselt werden.

Um die Abscanngeschwindigkeit zu erhöhen, ohne auf eine hohe Genauigkeit verzichten zu müssen, wurde schließlich von der DE 101 57 523 C1 vorgeschlagen, einen Kollimator mit mehreren verschieden großen Kollimatoröffnungen vorzusehen, die wahlweise in den Strahlengang bringbar sind. Dies erfolgte vorzugsweise mittels eines revolverähnlichen Mechanismuses, der eine runde Platte mit den verschieden großen Öffnungen dreht. Bei den heute für die Therapie gebräuchlichen energiereichen Strahlen ist jedoch zur Abschirmung eine Materialstärke von 6 bis 10 cm erforderlich. Auf diese Weise erhält man entweder einen Kollimator mit sehr hohem Gewicht, oder man muß sich auf wenige, beispielsweise drei Öffnungsgrößen beschränken. Aber selbst bei einer solchen Beschränkung ist es erforderlich, die nicht verwendeten Öffnungen abzudecken, damit nicht Bereiche entstehen, welche nur mit unzureichender Materialdicke abgeschirmt sind. Es ist also außer der Platte mit den Öffnungen noch eine Abschirmplatte erforderlich, welche ebenfalls mehrere Zentimeter Stärke aufweisen muß. Aus diesem Grund wird der Kollimator relativ schwer, was den Aufwand an Führungen und Antrieben entsprechend vergrößert. Ein weiterer Nachteil dieses Kollimators besteht darin, daß aus den oben genannten Gründen nur wenige festgelegte Kollimatoröffnungen verfügbar sind, wodurch die Variabilität der Strahlbegrenzung stark limitiert ist. Insbesondere ist es auch wegen der Beschränkung auf wenige Öffnungen nicht möglich, große Öffnungen verschiedener Durchmesser vorzusehen, mit denen zunächst ein Bereich der zu bestrahlenden Fläche behandelt werden kann, der möglichst groß ist, um danach noch die Randbereiche mit abgestuft feineren Strahlenbündeln zu behandeln. Da die Verweilzeit einer Strahlenapplikation für jeden Punkt einer Fläche mehrere Sekunden ausmacht, bedeutet dies, daß ein Abscannen einer Fläche mit festgelegten Größen von Strahlenbündeln zeitaufwendiger ist als mit optimal einstellbaren Größen. Dies ist insbesondere dann der Fall, wenn die Strahlenbündel schmäler sind, als dies bezüglich der Bestrahlungsfläche möglich wäre. Dadurch wird die Gesamtbehandlungsdauer verlängert. Dies ist nicht nur für den Patienten, der fixiert werden muß, unangenehm, sondern senkt auch die Anzahl der an einem Gerät möglichen Behandlungen, was in Anbetracht hoher Anschaffungs- und Betriebskosten solcher Geräte von hoher wirtschaftlicher Bedeutung ist. Außerdem ist die Genauigkeit der Randbereicherfassung begrenzt, was in Bereichen wie angrenzende Nerven kritisch ist.

Schließlich wurde von der EP 0 382 560 A1 ein Bestrahlungsgerät der eingangs genannten Art vorgeschlagen. Die Schrift befaßt sich hauptsächlich mit einer Kombination von Bildgebung und Bestrahlungsbehandlung. Unter anderem wird ein Kollimator mit einer Irisblende vorgeschlagen, wodurch die Blendenöffnung sehr variabel gestaltet werden kann. Dem Strahl wird jedoch durch die Irisblende ein Querschnitt von einem Vieleck aufgeprägt. Erfolgt nun eine Applikation aus verschiedenen Richtungen, so erhält diese vieleckige Querschnittsfläche, beispielsweise in Sechseckform, ständig eine neue Orientierung im Raum, welche in die Berechnung der Applikation nach Raumwinkel, Blendenöffnung und Bestrahlungsdauer mit einbezogen werden müßte. Dies würde die Berechnung vieler vorzunehmender Einzelbestrahlungen - dies sind bei einer einzigen Bestrahlungsbehandlung oft mehr als hundert - nach den vorgenannten Parametern mit dem Ziel, ein bestimmtes räumliches Bestrahlungsprofil zu erzielen, erheblich komplizierter machen. Würde man die unterschiedlichen Orientierungen der Vieleckform des Strahls in Kauf nehmen, ohne sie zu eliminieren, so würde dies zu Fehlern im applizierten Bestrahlungsprofil führen. Diese Fehler wären um so größer, je mehr ein Vieleck von der Kreisform abweicht. Natürlich könnte eine Irisblende auch mit mehr Blendenblättern ausgestattet werden, so daß das Vieleck ohne allzu große Ungenauigkeit als Kreis in die Berechnung einbezogen werden kann, was diese erheblich vereinfacht, so daß Rechnerkapazität und/oder Berechnungszeit in einem vertretbaren Rahmen bleiben. Die Folge wäre jedoch bei der Unterbringung in einer Blende ein hoher mechanischer Aufwand, der ab einer gewissen Zahl von Blendenblättern mit Antrieb und Führung eines jeden Blendenblattes auch an die Grenze der räumlichen Unterbringung stößt.

Hinzu kommen jedoch bei der durch die EP 0 382 560 A1 vorgeschlagenen Lösung noch weitere Probleme:

Um die heute gebräuchlichen energiereichen Therapiestrahlen, deren Leistung im Megavoltbereich liegt, abzuschirmen, sind Materialstärken des Abschirmungsmaterials, meist Wolfram, von 6 bis 10 cm erforderlich. Blendenblätter dieser Dicke können, vor allem, wenn möglichst viele eingesetzt werden sollen, nicht mehrfach übereinandergeschichtet werden, sondern es ist erforderlich, daß sie aneinanderliegende Seitenflächen aufweisen, wie dies von vielen der vorgenannten Schriften vorgeschlagen wurde. Dadurch entsteht auch bei feinster Bearbeitung der Seitenflächen ein geringer Spalt, welcher hindurchdringende Leckstrahlen zur Folge hat. Dem kann man zwar abhelfen, wenn man die Spalte überdeckende Laschen vorsieht, wie dies die DE 20 53 089 A vorschlägt, was jedoch insbesondere bei vielen Blendenblättern die Kompliziertheit erhöht und weiteren Einbauraum benötigt, da für eine ausreichende Abschirmung auch die Laschen entsprechend dick sein müssen.

Insbesondere wird jedoch bei der Verwendung einer solchen Irisblende deren großer Vorteil, nämlich daß auch Strahlenbündel mit unterschiedlicher Querschnittsfläche gebildet werden können - was die Gesamtbestrahlungszeit wesentlich verkürzt -, andererseits wiederum zum Nachteil: Die strahlbegrenzenden Flächen der Irisblende sind je nach Blendenöffnung gleichzeitig auch Gleitflächen zwischen den Blendenblättern. Dies bedeutet, daß sie im wesentlichen senkrecht zum Strahlengang oder in geringer Abweichung davon verlaufen müssen. Bei Abschirmungen und damit einer Dicke der Blendenblätter in einem Bereich von 6 bis 10 cm verlaufen die Strahlen bei weit geöffneter Irisblende in den äußeren Bereichen des Strahlenbündels in nicht unerheblichem Maß derart divergierend, daß sie nur teilweise abgeschirmt werden, da sie teilweise außerhalb des Materials verlaufen. Es entsteht also um die voll durch die Blende hindurchlaufenden Strahlen eine Art Hof, also der oben bereits erwähnte Halbschatten, bis dann der Bereich der vollen Abschirmung kommt. Es muß somit bei einer Bestrahlungseinrichtung der eingangs genannten Art entweder eine größere Mitbestrahlung umliegenden Gewebes in Kauf genommen werden - was aber aus medizinischer Sicht unakzeptabel ist - oder es müssen die Randbereiche mit einem sehr feinen Strahl, der auch wenig Halbschatten aufweist, abgescannt werden und zwar entsprechend abgestuft um den durch den großen Strahl applizierten Halbschatten möglichst exakt wieder zu eliminieren. Auch dadurch würde der Rechen- und Zeitaufwand vervielfacht.

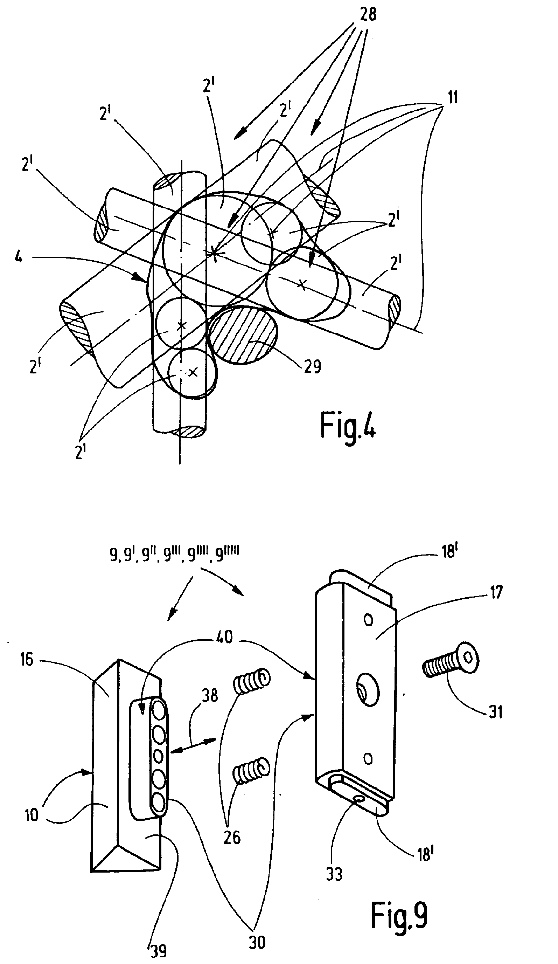

Der größte Nachteil des Bestrahlungsgeräts der EP 0 382 560 A1 besteht jedoch darin, daß neben dem Einsatz eines Röntgenstrahlscanners für die Bilderzeugung zwar auch eine Art "Scannbewegung" des Therapiestrahls erwähnt ist, die allerdings nur dahingehend konkretisiert wird, daß versucht wird, aus jedem Raumwinkel eine annähernd dem Behandlungsobjekt entsprechende Form des Strahlenquerschnitts mittels der Irisblende zu erzielen. Gelehrt wird somit lediglich eine Übereinanderlagerung von Einzelapplikationen, die aus verschiedenen Raumwinkeln erfolgen, jedoch nicht ein Aneinanderfügen von Einzelapplikationen, wie bei dem Scannen, das die DE 199 22 656 A1 und die DE 101 57 523 C1 lehren. Dies ermöglicht zwar neben der Ausbildung runder Bestrahlungsräume auch die Ausbildung von Bestrahlungsräumen mit elliptischem Querschnitt (wie dies in Fig. 4 der oben genannten Schrift ersichtlich ist), unregelmäßige Räume, die für die Behandlungsobjekte die Regel sind, lassen sich so allerdings nicht aus Einzelapplikationen zusammensetzen.

Der Erfindung liegt daher die Aufgabe zugrunde, ein Bestrahlungsgerät und einen Kollimator der eingangs genannten Art derart weiterzubilden, daß mit möglichst geringem mechanischem, Rechen- und Bestrahlungszeitaufwand ein räumliches Bestrahlungsprofil möglichst exakt applizierbar ist und deshalb umliegendes Gewebe maximal geschont wird.

Die Aufgabe wird erfindungsgemäß dadurch gelöst, daß sich mindestens eine weitere Irisblende gleichachsig im Strahlengang befindet, wobei die Irisblenden derart angeordnet sind, daß die jeweiligen Blendenblätter der Blenden derart gegeneinander in Drehrichtung um die Achse versetzt angeordnet sind, daß der durch den Kollimator begrenzte Strahl den Querschnitt eines Vielecks hat, dessen Eckenzahl der Zahl der Blendenblätter aller Irisblenden entspricht, daß die Blendenblätter gleiche Winkel einschließende, aneinanderliegende Seitenflächen aufweisen, wobei die Stellbewegungen der Blendenblätter auf geradlinige Weise senkrecht zur Winkelhalbierenden der mit den angrenzenden Blendenblättern in Eingriff befindlichen Seitenflächen verlaufen, und daß die Steuerung derart ausgebildet ist, daß sie die Bestrahlung eines unregelmäßigen Raums durch Überlagerung und Aneinanderfügen vieler bestrahlter Räume bewirken kann.

Bezüglich des Kollimators wird die Aufgabe erfindungsgemäß dadurch gelöst, daß sich mindestens eine weitere Irisblende gleichachsig im Strahlengang befindet, wobei die Irisblenden derart angeordnet sind, daß die jeweiligen Blendenblätter der Blenden derart gegeneinander in Drehrichtung um ihre Achse versetzt sind, daß der durch den Kollimator begrenzte Strahl den Querschnitt eines Vielecks hat, dessen Eckenzahl der Zahl der Blendenblätter aller Irisblenden entspricht, und daß die Blendenblätter gleiche Winkel einschließende, aneinanderliegende Seitenflächen aufweisen, wobei die Stellbewegungen der Blendenblätter auf geradlinige Weise senkrecht zur Winkelhalbierenden der mit den angrenzenden Blendenblättern in Eingriff befindlichen Seitenflächen verlaufen.

Bezüglich der Weiterbildungen des Kollimators wird auf die Beschreibung der Bestrahlungsvorrichtung verwiesen, soweit sich diese mit dem Kollimator beschäftigt.

Die Erfindung geht von einer Bestrahlungsvorrichtung aus, mit der aus unterschiedlichen Richtungen Strahlenapplikationen erfolgen, um das zu bestrahlende Gewebe wesentlich intensiver zu bestrahlen als das umliegende Gewebe. So kann letzteres geschont und das kranke Gewebe zerstört werden. Die Erfindung geht weiterhin von einer Bestrahlungsvorrichtung und einem entsprechenden Kollimator aus, bei der der Kollimator die Form des Behandlungsobjekts nicht nachbildet, sondern eine Art räumliches Abscannen erfolgt, bei dem allerdings zur Bestrahlungszeitverkürzung unterschiedlich große Strahlenbündel eingesetzt werden. So können größere Bereiche des Behandlungsobjekts mit größeren Strahlenbündeln behandelt werden, um danach mit zunehmend kleineren Strahlenbündeln eine exakte Nachbildung des Behandlungsobjekts vorzunehmen. Die Strahlenbündel können dabei aus verschiedenen Richtungen jeweils feststehend eine vorbestimmte Zeit appliziert werden oder die bestrahlte Fläche kann eine Bewegung über das Behandlungsobjekt vollziehen. Dabei kommt es nicht nur zu einem Überlagern vieler bestrahlter Räume, sondern auch zu einem Aneinanderfügen derselben oder einem Aneinanderfügen mit teilweise Überlagerung, wodurch ein Dosierungsprofil in einer für die Behandlung erforderlichen Intensität eines unregelmäßigen Raums erzielbar ist. So wird ein räumliches Bestrahlungsprofil erzielt, wobei - soweit medizinisch angesagt - auch verschiedene Intensitätsprofile applizierbar sind. Kritische angrenzende Bereiche, wie Nerven, können dabei auch völlig ausgespart werden.

Ein wichtiges Element der Erfindung, sowohl bezüglich der Bestrahlungsvorrichtung, als auch bezüglich des Kollimators, ist, daß sich zum Zweck der einstellbaren Strahlbegrenzung gleichachsig mindestens zwei Irisblenden im Strahlengang befinden. Dieses Element erfordert dann einige Ausgestaltungen, um die in der Beschreibungseinleitung erwähnten technischen Probleme des vorbekannten Standes der Technik zu lösen:

Zunächst ist natürlich erforderlich, daß die Irisblenden Blendenblätter mit gleichem Winkel einschließende, aneinanderliegenden Seitenflächen aufweisen. Dadurch können überhaupt erst Irisblenden mit den entsprechenden Abschirmungsstärken gebaut werden. Die für diese Strahlen erforderlichen Abschirmungsstärken betragen 6-10 cm üblichen Abschirmungsmaterials, wie beispielsweise Wolfram. Selbst, wenn man dieses auf mehrere Irisblenden verteilt, sind die Blendenblätter immer noch zu dick, um sie überlappend aufeinanderschichten zu können.

Gerade wenn eine Vielzahl von Einzelapplikationen aus unterschiedlichen Richtungen erfolgen soll, aber auch dann, wenn man langsam über das Behandlungsobjekt wandernde Strahlenbündel einsetzt, ist diese Applizierung eines vorgegebenen räumlichen Bestrahlungsprofils nur mit unvertretbar großem Rechenaufwand umsetzbar, wenn die Strahlenbündel Vielecke mit relativ wenigen Ecken aufweisen, wie beispielsweise eine Sechseckform, da dann diese Vieleckform und deren ständig wechselnde Ausrichtung in diese Berechnung einbezogen werden müßte. Darum besteht ein wesentliches Merkmal der Erfindung darin, daß die Blenden der angeordneten Irisblenden derart gegeneinander in Drehrichtung versetzt angeordnet sind, daß der durch den Kollimator begrenzte Strahl den Querschnitt eines Vielecks hat, dessen Eckenzahl der Zahl der Blendenblätter aller Kollimatoren entspricht. Dadurch ist es möglich, Strahlen durch Vielecke zu begrenzen, die sich stark der Kreisform annähern und deshalb als Kreis in die Berechnung einbezogen werden können, ohne eine aus medizinischer Sicht untolerierbare Abweichung vom vorgegebenen Bestrahlungsprofil zur Folge zu haben. Durch zwei Irisblenden erhält man ein Vieleck, das die doppelte Eckenzahl aufweist als jede Irisblende für sich, bei mehr Irisblenden entsprechend mehr. Dadurch ist es möglich, eine Vieleckform mit hoher Eckenzahl zu erhalten, ohne daß man Irisblenden mit allzu hoher Blattzahl benötigt, die bezüglich der Mechanik, insbesondere der Führungen und des Antriebs räumlich schwer unterzubringen sind. Außerdem ist die Herstellung einer größeren Zahl einfacher aufgebauter Irisblenden preisgünstiger als eine kleinere Anzahl wesentlich komplizierter Irisblenden. Eine bevorzugte Ausführungsforrn wäre dabei die Anordnung zweier sechsblättriger Irisblenden, womit eine zwölfeckige Strahlbegrenzung erzielbar ist, die für den genannten Zweck in der Regel ausreicht. Selbstverständlich könnte beispielsweise durch drei solcher Irisblenden auch eine 18-eckige-Strahlbegrenzung oder in entsprechender Weise mehr erzielt werden.

Mit dieser Anordnung wird jedoch noch gleichzeitig das Problem der Leckstrahlung gelöst: Bei Irisblenden der genannten Art entsteht - wie einleitend bereits erörtert - das Problem, daß an den Aneinandergrenzungen der Blendenblätter immer mehr oder weniger große Spalte entstehen, da ein absolutes Aneinanderliegen von Flächen praktisch nicht herstellbar ist. In der Regel liegt aber die Grenze absoluten Aneinanderliegens der Flächen schon vor dem herstellungstechnisch möglichen, da ein Kaltverschweißen ebenfalls ausgeschlossen werden muß. Im übrigen muß sich auch der Herstellungsaufwand in bezahlbaren Grenzen halten. Dadurch, daß die Blendenblätter der Irisblenden in Drehrichtung versetzt sind, werden durch solche Spalte gehende Leckstrahlen durch die weitere oder die weiteren Irisblenden aufgefangen. Da diese Leckstrahlen wegen der geringen Spalte nicht sehr groß sind, ist die Hälfte der Abschirmdicke für die Abschirmung dieser Leckstrahlen auch ausreichend. Bei drei Irisblenden würde man sogar zwei Drittel der Gesamtabschirmdicke erhalten.

Die Strahlen der Strahlungsquelle sind jedoch nicht nur allseitig auf das Behandlungsobjekt auszurichten, wie das bei Kollimatoren der Fall ist, welche die Form des Tumors mehr oder weniger exakt nachbilden, beispielsweise bei der EP 0 382 560 A1, sondern es muß außerdem ein räumliches Bestrahlungsprofil gebildet werden, indem viele Bestrahlungsbereiche verschiedener Größen in überdeckender, in aneinanderreihender und dabei auch oft teilweise überdeckender Weise zusammengefügt werden. Dazu ist eine Einrichtung erforderlich, welche die Strahlungsquelle mit dem Kollimator aus mehreren Irisblenden kurzzeitig in eine Vielzahl von Positionen im Raum bringt, welche sowohl eine Anordnung im Raum, als auch eine Ausrichtung in einem bestimmten Raumwinkel beinhalten. Dies kann schrittweise oder kontinuierlich erfolgen. Ein derartiger aus Strahlungsquelle und Abschirmung, sowie insbesondere dem Kollimator bestehender Bestrahlungskopf, muß also bezüglich Gewicht und Größe so klein wie möglich gehalten werden. Dies bedeutet, daß komplizierte Stellvorgänge der Blendenblätter durch kombinierte Dreh- und Schiebebewegungen, wie sie von der FR 2 524 690 A vorgeschlagen werden, keine gerätetechnisch und wirtschaftlich sinnvolle Lösung darstellen.

Deshalb sieht die Erfindung vor, daß die Blendenblätter gleiche Winkel einschließende, aneinanderliegende Seitenflächen aufweisen, wobei die Stellbewegungen der Blendenblätter ausschließlich geradlinig senkrecht zur Winkelhalbierenden der mit den angrenzenden Blendenblättern in Eingriff befindlichen Seitenflächen verlaufen. Dies hat den Vorteil einer einfachen Bewegung eines jeden Blendenblattes einer Irisblende auf einer geraden Führung, wobei diese Bewegungen auch noch für alle Blendenblätter einer Irisblende synchron erfolgt. Für die weitere oder die weiteren Irisblenden erfolgen dann gleichzeitig Stellbewegungen der Blendenblätter. Dadurch können Antrieb und Stellmechanik einfach ausgebildet werden und die Führungen sind einfache Linearführungen. Dies ermöglicht es vor allem, Antrieb, Führungen und Stellmechanik in einem Bestrahlungskopf derart unterzubringen, daß dieser noch leicht und klein genug ist, um seinerseits mit der oben genannten Einrichtung ohne allzu hohen Aufwand in die verschiedenen Raumpositionen und Ausrichtungen gebracht zu werden, was für die erfindungsgemäße Vorrichtung unabdingbar ist.

Auf diese Weise entsteht eine Bestrahlungsvorrichtung, die bei vertretbarer Baugröße und vertretbarem Gewicht die vorgenannte Aufgabe löst, wobei Baugröße und Gewicht wiederum eine Rückwirkung auf die Größe des Antriebs und die erforderliche Antriebsenergie sowie die Stellgeschwindigkeiten hat. Auf diese Weise kann neben der Reduzierung des gerätetechnischen Aufwands sowie des Aufwands an Rechnerhardware und Software auch Gewicht und Energie eingespart werden. Vor allem werden die für die Applikationen der Bestrahlung nicht nutzbaren Einstell- und Rechenzeiten und damit die Dauer einer Behandlung reduziert. Diese Vorteile konnten durch die Erfindung mit einer größtmöglichen Schonung des gesunden Gewebes bei einer Strahlenapplikation verknüpft werden, so daß auch die Risiken für Patienten weiter verringert werden können.

Die Aufteilung der notwendigen Dicke des Abschirmmaterials auf mehrere Irisblenden hat außerdem den Vorteil, daß sich die Bauhöhe der einzelnen Blendenblätter entsprechend reduziert und dadurch diese besser in den Linearführungen geführt werden können, wobei die Aneinandergrenzungen der Seitenflächen der Blendenblätter verbessert und insbesondere die Gefahr eines unparallelen Aneinanderliegens durch ein Verkippen der Blendenblätter verringert wird.

Die vorgenannte gleichachsige Anordnung von Irisblenden ermöglicht es auch, auf einfachste Weise die Halbschatten zu verringern, indem die Stellmittel der Irisblende derart ausgebildet sind, daß die jeweilige Blendenöffnung innerhalb des gesamten Stellbereichs der Divergenz der Strahlen, bei deren Begrenzung Rechnung tragen. Auf diese Weise wird die Abschirmung entsprechend der Divergenz der Strahlen gestuft. Dabei sind die Stellwege der Blendenblätter bezüglich der einen Kollimator bildenden mehreren Irisblenden bei der oder den näher an der Strahlenquelle liegenden Irisblenden kürzer als die Stellwege der Blendenblätter der weiter weg liegenden Irisblende beziehungsweise Irisblenden. Diese Maßnahme ist jedoch nur dann erforderlich, wenn Blendenöffnungen so groß sind, daß eine ins Gewicht fallende Divergenz der Strahlen auftritt. Divergenz der Strahlen und mögliche Halbschatten sind bei sehr kleinen Blendenöffnungen vernachlässigbar. Diese sind daher auch geeignet, um an besonders kritischen Grenzbereichen, wie beispielsweise angrenzenden Nerven, ganz exakte Grenzen des bestrahlten Bereichs zu erzeugen, nachdem zuvor die großräumigen Bereiche mit breiteren Strahlen behandelt wurden.

Vorzugsweise ist der Kollimator derart ausgebildet, daß durch die gleiche Blendenblattzahl der mindestens zwei Irisblenden, durch einen entsprechenden Versatz derselben in Drehrichtung um ihre Achse und durch die Ausbildung der Stellmittel der Irisblenden ein Querschnitt eines gleichseitigen Vielecks gebildet wird, da ein solches der Kreisform am nächsten kommt. Gleiche Stellwege für die Blendenblätter der jeweiligen Irisblende und eine entsprechende Abstimmung der Stellwege der verschiedenen Irisblenden auf die Strahlendivergenz im gesamten Einstellbereich dienen dazu, daß die Strahlen dieses gleichseitige Vieleck unabhängig von dessen Größe bilden.

Vorzugsweise ist die Steuerung derart ausgebildet, daß aufgrund von verschiedenen Blendenöffnungen, Positionierungen von Strahlungsquelle und Kollimator im Raum und der Ausrichtung in bestimmten Raumwinkeln durch eine Zusammenfügung einer Vielzahl von lediglich begrenzte Teilräume des Behandlungsobjektes erfassenden Einzelapplikationen mit einem im wesentlichen runden Strahlquerschnitt eine unregelmäßige Raumform des Behandlungsobjektes nachgebildet werden kann, um sie mit relativ exakten Grenzen in vorgegebener Intensität wesentlich höher zu bestrahlen als das umliegende Gewebe. Es wird dabei mit einer von der Form des Behandlungsobjektes in der Regel erheblich abweichenden Gestalt der behandelten Teilräume eine Art räumliches Abscannen vorgenommen, wobei gegenüber dem bekannten Abscannen einer Fläche, was meist zeilenweise über eine Fläche mit gleich dickem Strahl vorgenommen wird, dies in Beziehung auf einen Raum vorgenommen und dabei Strahlquerschnitte verschiedener Größen eingesetzt werden, wobei auch nicht zeilenweise, sondern nach vorheriger Berechnung viele Strahlen unterschiedlicher Größe räumlich ineinandergeschachtelt werden. Dieses Ineinanderschachteln betrifft sowohl eine Nebeneinander- als auch eine Übereinanderlagerung einschließlich teilweiser Neben- und Übereinanderlagerung der Teilräume der Einzelapplikationen. Durch die verschiedenen Größen der Räume der Einzelapplikationen ist es möglich, große Bereiche schnell zu bestrahlen und die verbleibenden Bereiche mit zunehmender Feinarbeit zu behandeln, bis die fast immer unregelmäßige Raumform des Behandlungsobjekts mit der vorgegebenen Intensität bestrahlt ist. Dabei können auch unregelmäßige Raumformen unterschiedlicher Intensität erzeugt werden.

Vorzugsweise wird diese Strahlenapplikation derart vorgenommen, daß die Einzelapplikationen jeweils in einem gewissen Zeitraum mit unveränderten Parametern erfolgen. Der Vorteil besteht darin, daß während der Applikation keine mechanischen Stellvorgänge ablaufen, wodurch sich besser die erforderlichen Toleranzen einhalten lassen.

Man kann sich die Vornahme der Applikation so vorstellen, daß mit jeder Einzelapplikation jeweils ein zylindrischer Bereich erfaßt wird, der durch den ganzen menschlichen Körper geht. Diese zylindrischen Bereiche, die auch noch verschiedene Größen aufweisen, kommen dabei aus allen möglichen Richtungen des Raumes und kreuzen sich im Bereich des Behandlungsobjekts derart, daß dort ein vielfaches der Strahlungsdosis zur Wirkung kommt, wie bei dem umliegenden Gewebe. Es verhält sich somit wie bei einer Anfüllung eines Raumes mittels einer Vielzahl verschieden großer Zylinder, die nicht nur in allen möglichen räumlichen Ausrichtungen liegen, sondern dabei einen Raum auch noch vielfach belegen. Gerade durch diese Kombination von Nebeneinanderlagerungen, vielfachen Übereinanderlagerungen und Kombinationen von beidem im Bereich des Behandlungsobjekts kommt es dort zu der erforderlichen Steigerung der Bestrahlungsintensität mit dem Ziel, daß das umliegende Gewebe möglichst unbeeinträchtigt ist, beziehungsweise sich zumindest wieder regenerieren kann, das zu behandelnde Gewebe, beispielsweise der Tumor, jedoch abstirbt. In entsprechender Weise ist es auch möglich, kritische angrenzende Bereiche wie Nerven am Randes des Behandlungsobjekts auszusparen, derart daß dort möglichst keine direkte Strahlung hindurchgeht.

Eine besonders zweckmäßige Ausgestaltung der Erfindung betrifft den Aufbau der Irisblenden. Dabei wird ein besonders gutes Aneinanderliegen der Blendenblätter dadurch erreicht, daß Kraftbeaufschlagungen vorgesehen sind, die die Seitenflächen der Blendenblätter gegeneinanderdrücken. Dies kann beispielsweise dadurch erreicht werden, daß die Blendenblätter aus einem Abschirmelement aus Abschirmmaterial und einem Lagerelement bestehen, wobei das Lagerelement einen Führungsteil einer Linearführung für die Stellbewegung aufweist und das Abschirmelement auf dem Lagerelement derart federgelagert ist, daß es gleichmäßig in Richtung der angrenzenden Blendenblätter gedrückt wird. Auf diese Weise werden zahlreiche Vorteile erreicht: Es kommt immer zu einem exakten Flächenkontakt, da die Seitenflächen immer plan aufeinanderliegen, so daß eventuelle Spalte nur noch durch Flächenunebenheiten oder Oberflächenrauheiten entstehen können. Dieser Flächenkontakt ist auch dann garantiert, wenn gewisse Maßabweichungen vorhanden sind, so daß an die Exaktheit der Fertigung keine Anforderungen gestellt werden müssen, die den Preis erheblich in die Höhe treiben würden. Dies verbilligt die Bestrahlungsvorrichtung beträchtlich. Außerdem ist dadurch auch ein Klemmen von Blenden und/oder Stellmechanismen weitgehend ausgeschlossen, auch wenn gewisse Toleranzabweichungen vorhanden sind. Ein solches Klemmen kann auch nachträglich kaum eintreten, beispielsweise durch Verschleiß, Schmutz, kleine Beschädigungen oder Wärmeausdehnung. Dadurch wird die Vorrichtung nicht nur preiswerter herstellbar, sondern gleichzeitig auch wesentlich funktionssicherer.

Der Bestrahlungskopf, welcher die Strahlungsquelle und den Kollimator enthält, kann auf einer Gantry angeordnet sein, um auf diese Weise das Behandlungsobjekt aus verschiedenen Richtungen bestrahlen zu können. Vorzugsweise wird der Bestrahlungskopf, der die Strahlungsquelle und den Kollimator enthält, jedoch auf einem Roboterarm angeordnet, der derart ausgebildet ist, daß er den Bestrahlungskopf in jede Raumposition und jede Raumwinkelausrichtung bringen kann. Dies hat gegenüber der bekannten Anordnung von Bestrahlungsköpfen auf einer Gantry den Vorteil, daß eine wesentlich vielfältigere Positionierung und Ausrichtung im Raum möglich ist. Bei einer Gantry kreist der Bestrahlungskopf um ein Zentrum, in dem das Behandlungsobjekt positioniert wird. Weitere Variationen sind lediglich dadurch möglich, daß auch die Position des Patienten etwas verändert wird, so daß sich das Zentrum an verschiedenen Stellen des Behandlungsobjekts befinden kann. Dagegen ist bei einem Roboterarm eine Ausrichtung auf ein Zentrum überhaupt nicht erforderlich, da der Roboterarm beliebige Raumkoordinaten und beliebige Ausrichtungen im Raum ansteuern und beliebig miteinander kombinieren kann, die einzige, aber gegenüber einer Gantry wesentlich geringeren Limitierung der Positionierungsmöglichkeiten, ist die jeweilige Konstruktion des Roboterarms. Es sind somit wesentlich mehr Freiheitsgrade in der Einstellung möglich, wodurch das oben beschriebene vielfältige Nebeneinander-, Übereinander-und Ineinanderschachteln unterschiedlich großer Teilräume der Einzelbehandlungen erst in optimaler Weise möglich ist und sich dabei auch kritische Bereiche, wie Nerven, besser aussparen lassen.

Um Schwenkbewegungen der Strahlausrichtung sehr schnell und äußerst exakt vornehmen zu können, kann vorgesehen sein, daß der Kollimator für Schwenkbewegungen in einem begrenzten Raumwinkelbereich auf einer kugeloberflächenförmigen Bahn allseitig verschiebbar gelagert ist, wobei die Achse immer auf die Strahlungsquelle ausgerichtet ist. Eine Lagerung eines Kollimators auf einer derartigen kugeloberflächenförmigen Bahn ist in der DE 101 57 523 C1 offenbart, darauf wird hiermit verwiesen. Selbstverständlich ist diese mit einer Gantry oder einem Roboterarm kombinierbar, so daß Stellbewegungen in einem kleineren Bereich mit dieser Bahn und in einem größeren Bereich mit der Gantry oder dem Roboterarm vorgenommen werden können.

Die Bestrahlungsvorrichtung kann derart ausgebildet sein, daß jede Irisblende über einen Antrieb verfügt. Dann kann die Steuerung derart ausgebildet sein, daß sie die jeweilige Blendenöffnung entsprechend der Divergenz der Strahlen steuert. Dies kann dann zweckmäßig sein, wenn der Kollimator mit den Irisblenden für den Einbau in verschiedene Bestrahlungsgeräte geliefert wird und die Abstände zur Strahlungsquelle unterschiedlich sind. Es kann dann durch die Steuerung eingestellt werden, wie sich die Blendenöffnungen zueinander verhalten. Es ist somit durch Programmierung möglich, der jeweiligen unterschiedlichen Divergenz der Strahlen Rechnung zu tragen. Alternativ können die Irisblenden auch mittels eines einzigen Antriebs betätigbar sein. Dann kann die Stellmechanik die jeweilige Blendenöffnung entsprechend der Divergenz der Strahlen bewirken. Dies ist weniger aufwendig und Fehlprogrammierungen sind diesbezüglich nicht möglich. Diese Alternative ist besonders dann zweckmäßig, wenn der Kollimator mit den Irisblenden in einer Bestrahlungsvorrichtung einer Strahlungsquelle fest zugeordnet ist.

Eine Ausführungsform sieht vor, daß die Blendenblätter einer jeden Irisblende in mindestens einer ein Führungsteil der Linearführung enthaltenden Führungsplatte geführt sind. Der andere Führungsteil der Linearführung befindet sich dann an den Blendenblättern, beispielsweise an den oben genannten Lagerelementen. Zur Betätigung der Blendenblätter kann vorgesehen sein, daß eine Kurvenscheibe mit den Blendenblättern in Wirkverbindung steht und daß der Antrieb die Stellbewegungen der Blendenblätter über eine relative Drehbewegung zwischen Führungsplatte und Kurvenscheibe bewirkt. Auf diese Weise wird durch eine Stellbewegung der Kurvenscheibe eine simultane Stellbewegung aller Blendenblätter erzeugt. In der Regel wird man dann die Führungsplatte feststehend anordnen und den Antrieb der Kurvenscheibe zur Drehung derselben zuordnen.

Um jegliche Kippbewegung der Blendenblätter mit Sicherheit auszuschließen, ist es zweckmäßig, wenn die Blendenblätter einer jeden Irisblende auch an der gegenüberliegenden Seite mittels einer weiteren Führungsplatte geführt sind.

Sind zwei Irisblenden vorgesehen, so kann der Antrieb zwischen den zwei Irisblenden eingreifen, um von dort beide Irisblenden zu betätigen. Dies bedeutet natürlich, daß die Stellbewegung auf die Blendenblätter gegebenenfalls derart übersetzt wird, daß dabei entsprechend der Divergenz der Strahlen die Blendenöffnungen immer unterschiedlich groß sind. Beispielsweise läßt sich dies dadurch bewirken, daß sich aus unterschiedlichen Ausführungen der Kraftübertragungselemente unterschiedliche Hebellängen der Kraftübertragungen und damit unterschiedliche Stellwege für die Einstellung der Blendenblätter ergeben. Eine weitere Möglichkeit besteht darin, daß der Antrieb einer Irisblende zugeordnet ist und über Mitnehmer der Antrieb der weiteren Irisblende bewirkt wird, wobei die Kraftübertragungselemente, beispielsweise die Kurvenscheiben, für die Bewirkung von der Divergenz der Strahlen entsprechenden Blendenöffnungen ausgestaltet sind.

Es kann natürlich auch vorgesehen sein, daß die Stellmittel für mehrere Krafteinleitungen des Antriebs ausgebildet sind. Dann kann mindestens einer Krafteinleitung ein Getriebe zugeordnet sein, durch das ein der Divergenz der Strahlen entsprechendes Verhältnis der Blendenöffnungen bewirkt wird. Dies bietet die Möglichkeit, daß durch Austausch oder Verstellung eines Übertragungsglieds des Getriebes der Kollimator auf verschiedene Strahlendivergenzen eingestellt werden kann. Das ist besonders dann von Vorteil, wenn eine Bestrahlungsvorrichtung nachträglich mit einem erfindungsgemäßen Kollimator ausgestattet wird. Die Einstellung kann dann beispielsweise durch Austausch eines Zahnrades oder Zahnradpaares erfolgen.

Die Kurvenscheiben können sternförmig angeordnete Mitnehmerkurven aufweisen, die auf bolzenförmige Mitnehmer der Blendenblätter wirken. Letztere sind zweckmäßigerweise als Rollen ausgebildet, um die entsprechende Leichtgängigkeit zu erhalten.

Eine Ausführungsform der Bestrahlungseinrichtung sieht vor, daß zwei sechsblättrige, um 30° in Drehrichtung versetzte Irisblenden angeordnet sind. Auf diese Weise ergibt sich ein regelmäßiges Zwölfeck, das in der Regel der Kreisform nahe genug kommt, um bei der Berechnung der vielen durchzuführenden Einzelapplikationen rechnerisch von einem Kreisquerschnitt des jeweils bestrahlten Raums ausgehen zu können.

Natürlich sind dies nur einige vorzugsweise gewählte Ausgestaltungen, es könnten beispielsweise auch drei vierblättrige Irisblenden übereinander angeordnet werden, um auf diese Weise ebenfalls ein regelmäßiges Zwölfeck zu erhalten, oder es kann natürlich auch eine andere Blendenblattzahl und Anzahl von Irisblenden gewählt werden.

Die Erfindung wird nachfolgend anhand von in der Zeichnung dargestellten Prinzipskizzen und Ausführungsbeispielen erläutert. Es zeigen
- **Fig. 1**: eine schematische Darstellung der erfindungsgemäßen Bestrahlungsvorrichtung im Schnitt,
- **Fig. 2**: eine schematische Darstellung zur Erläuterung der Funktionsweise des erfindungsgemäßen Kollimators,
- **Fig. 3a, 3b und 3c**: eine Prinzipskizze zur Erläuterung der Funktion einer Irisblende wie sie die Erfindung vorsieht,
- **Fig. 4**: eine Prinzipdarstellung einer Strahlenapplikation durch die erfindungsgemäße Bestrahlungsvorrichtung,
- **Fig. 5**: ein Ausführungsbeispiel eines erfindungsgemäßen Kollimators in perspektivischer Darstellung,
- **Fig. 6**: eine Draufsicht auf den Kollimator gem. Fig. 5,
- **Fig. 7**: eine Irisblende mit Antrieb und Getriebe,
- **Fig. 8**: ein weiteres Ausführungsbeispiel des erfindungsgemäßen Kollimators mit einem Antrieb, der zwischen zwei Irisblenden angeordnet ist und
- **Fig. 9**: eine besonders vorteilhafte Ausgestaltung eines Blendenblattes für eine Irisblende des erfindungsgemäßen Kollimators.

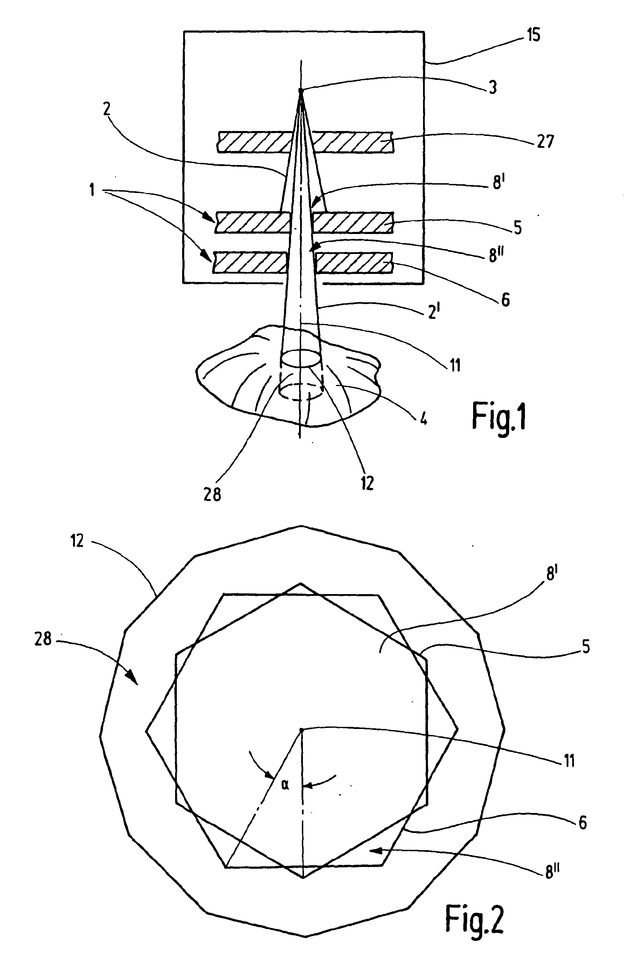

**Fig. 1** zeigt eine schematische Darstellung der erfindungsgemäßen Bestrahlungsvorrichtung im Schnitt. Dargestellt ist dabei der Bestrahlungskopf 15, welcher eine Strahlungsquelle 3 und den erfindungsgemäßen Kollimator 1 enthält. Nicht dargestellt ist die Einrichtung, welche dem Bestrahlungskopf 15 in verschiedene räumliche Positionen bewegt. Dabei kann es sich um eine auf diesem Gebiet bekannte Gantry handeln, welche den Bestrahlungskopf 15 auf ein Isozentrum ausrichtet, und so eine allseitige Bestrahlung eines Behandlungsobjektes 4, meistens eines Tumors, ermöglicht. Beim Einsatz einer solchen Gantry ist diese Ausrichtung auf ein Isozentrum durch diese Mechanik bedingt, Variationen sind lediglich dadurch möglich, daß der Patient und damit das Behandlungsobjekt 4 verschoben wird. Deshalb schlägt die Erfindung als eine zweckmäßige Ausgestaltung vor, den Bestrahlungskopf 15 an einem Roboterarm anzuordnen, so daß statt der Ausrichtung auf ein Isozentrum alle Koordinaten des Raums sowie alle Raumwinkelausrichtungen möglich sind, die die Mechanik des Roboterarms zuläßt. Es sind dabei wesentlich mehr Freiheitsgrade erzielbar und die verschiedenen Behandlungspositionen können wesentlich schneller angefahren werden.

Der Strahlungsquelle 3 ist zunächst in üblicher Weise ein Vorkollimator 27 zugeordnet, der dafür sorgt, daß die Strahlen 2 derart gebündelt sind, daß sie am Abschirmungsbereich des Kollimators 1 nicht vorbeigehen können. Das Kernstück der Erfindung ist schließlich der Kollimator 1, der aus mindestens zwei Irisblenden 5 und 6 besteht, welche bezüglich der Strahlungsquelle 3 gleichachsig angeordnet sind. Die Achse 11 ist strichpunktiert eingezeichnet, und es ist zu sehen, wie die Strahlen 2 durch den Kollimator 1 derart begrenzt werden, daß durch die begrenzten Strahlen 2' ein vorbestimmter bestrahlter Raum 28 im Bereich des Behandlungsobjektes 4 gebildet ist.

Dabei sind vorzugsweise die Blendenöffnungen 8', 8" der Irisblenden 5 und 6 derart unterschiedlich, daß sie der Divergenz der Strahlen (hier stark übertrieben gezeichnet) Rechnung tragen und so eine Begrenzung der Strahlen 2' schaffen, durch die der Querschnitt der Strahlen 2' ein Vieleck 12 ist, dessen Eckenzahl die Zahl der Blendenblätter aller Kollimatoren 5 und 6 entspricht. Letzteres wird zur Fig. 2 näher erläutert.

**Fig. 2** zeigt eine schematische Darstellung zur Erläuterung der Funktionsweise des erfindungsgemäßen Kollimators 1. Der Darstellung ist zugrunde gelegt, daß es sich bei den Irisblenden 5 und 6 um sechsblättrige Irisblenden 5, 6 handelt, so daß sechseckige Blendenöffnungen 8' und 8" entstehen. Durch einen Winkelversatz der Irisblenden 5, 6 in Drehrichtung um die Achse 11 um den Winkel α von 30° erhalten die Ecken des einen Sechsecks eine mittige Lage zu den Geraden des anderen Sechsecks. Auf diese Weise entsteht ein Vieleck 12, bei dem die Eckenzahl der Zahl der Blendenblätter aller Irisblenden entspricht. Wegen der Divergenz der Strahlen 2 ist die Blendenöffnung 8' der ersten Irisblende 5 so viel kleiner als die Blendenöffnung 8" der zweiten Irisblende 6, daß der Querschnitt der Strahlen 2' die Form eines regelmäßigen Zwölfecks 12 erhält. Dadurch erhält auch der bestrahlte Raum 28 eine zwölfeckige Form, so daß er dessen Querschnitt der Kreisform beziehungsweise die Raumform dem Zylinder derart nahekommt, daß er als letzteres in die Applikationsberechnung eingehen kann. Der Querschnitt des bestrahlten Raums 28 ist natürlich durch die Strahlendivergenz noch entsprechend größer als die Blendenöffnungen 8' und 8". Es sind die Blendenöffnungen 8' und 8" sowie das Vieleck 12 gegenüber den normalerweise eingesetzten Strahlen 2' vergrößert dargestellt.

Selbstverständlich ist es erfindungsgemäß möglich, statt der zwei Irisblenden 5 und 6 auch noch weitere Irisblenden vorzusehen, oder es ist auch denkbar, daß Irisblenden mit einer abweichenden Blattzahl verwendet werden. Dann muß selbstverständlich auch der Winkelversatz α anders gewählt werden, um wiederum ein gleichseitiges Vieleck 12 zu erreichen. Selbstverständlich sollte die Blendenblattzahl der Irisblenden gleich sein, da sonst ein gleichseitiges Vieleck 12 nicht erzielbar ist.

Die **Fig. 3a, 3b und 3c** zeigen eine Prinzipskizze zur Erläuterung der Funktion einer Irisblende 5 oder 6, wie sie in der Erfindung vorgesehen sind. Dabei zeigt die **Fig. 3a** die Irisblende 5 oder 6 in geschlossenem Zustand, die **Fig. 3b** mit einer kleinen Blendenöffnung 8 und die **Fig. 3c** mit einer maximalen Blendenöffnung 8.

Die dargestellte Irisblende 5 oder 6 verfügt über sechs Blendenblätter, 9, 9', 9", 9"', 9"" und 9""', wobei von diesen hier nur die Abschirmelemente 16 gezeichnet sind. Im geschlossenen Zustand liegen diese Blendenblätter 9, 9', 9", 9"', 9"", 9""' mit ihren aneinandergrenzenden Seitenflächen 10 derart aneinander, daß diese Seitenflächen 10 die Diagonalen des Sechsecks bilden, wie dies die **Fig.3a** zeigt. Soll eine solche Irisblende 5, 6 geöffnet werden, so müssen diese in **Fig. 3a** zusammenliegenden inneren Spitzen der Blendenblätter 9, 9', 9", 9"', 9"" und 9""' einen Stellweg 14 zurücklegen, der ausgehend von der Achse 11 nach außen verläuft, wie dies **Fig. 3c** für das Blendenblatt 9' zeigt. Ein solcher Stellweg 14 ist dadurch erzielbar, daß die Blendenblätter 9, 9', 9", 9"', 9"", 9""' eine geradlinige Bewegung in Richtung der Pfeile 13 vollziehen, also in einer Richtung, welche senkrecht zur in Fig. 3c strichpunktiert eingezeichneten Winkelhalbierenden 25 (in Bezug auf die Seitenflächen 10) verläuft. Dabei müssen selbstverständlich alle Blendenblätter 9, 9', 9", 9"', 9"", 9""' exakt simultan die gleiche geradlinige Stellbewegung 13 vollziehen. Eine beispielhafte Möglichkeit der praktischen Realisierung derartiger simultaner Stellbewegungen 13 sind den Ausführungsbeispielen zu entnehmen.

Erfindungswesentlich ist dabei die einfache geradlinige Stellbewegung 13, durch die eine Blendenöffnungseinstellung mit geringem Aufwand möglich ist. Beispielsweise kann dadurch eine Irisblende 5, 6 mittels eines Antriebs 19 betätigbar werden oder es ist sogar möglich, von einem Antrieb 19 ausgehend, alle Irisblenden 5, 6 in die richtige Blendenöffnungsposition zu bringen. Durch ein solches relativ einfaches Stellmittel 7 wird ein Bestrahlungskopf 15 verfügbar, der bezüglich Volumen und Gewicht noch gut durch einen Roboterarm handhabbar ist, der keine allzu große Baugröße aufzuweisen braucht. Dies ist insbesondere deshalb wichtig, weil man dadurch auf Roboterarme zurückgreifen kann, welche in anderen technischen Gebieten ihren Einsatz finden. Dadurch können teure Spezialanfertigungen vermieden werden und die Bestrahlungsvorrichtung ist relativ preisgünstig realisierbar.

**Fig. 4** zeigt eine Prinzipdarstellung einer Strahlenapplikation durch die erfindungsgemäße Bestrahlungsvorrichtung. Für die Arbeitsweise der Bestrahlungsvorrichtung ist entscheidend, daß durch den Kollimator 1 die Strahlen 2' nicht derart begrenzt werden, daß der bestrahlte Raum 28 durch die Begrenzung bezüglich seiner Außenkontur der Außenkontur des Behandlungsobjektes 4, also beispielsweise des Tumors, entspricht, sondern daß die Außenkontur des Behandlungsobjektes 4 aus der Zusammensetzung der bestrahlten Räume 28 vieler Einzelapplikationen erzielt wird, wobei bei jeder Applikation das durch den Kollimator 1 begrenzte Strahlenbündel 2' einen Querschnitt als Vieleck 12 aufweist, der im wesentlichen der Kreisform entspricht. Dabei zeigt die Fig. 4 in stark vereinfachter Darstellung, wie derartige Einzelapplikationen zusammengesetzt werden können. Dargestellt ist ein Behandlungsobjekt 4, beispielsweise ein Tumor, der sich in unmittelbarer Nähe eines kritischen Gewebes, beispielsweise eines Nervs 29 befindet. In einem solchen Fall muß eine Applikation der Strahlen 2' derart vorgenommen werden, daß das Behandlungsobjekt 4 eine maximale Strahlendosis erhält, das umliegende Gewebe eine möglichst geringe Strahlendosis und das kritische Gewebe 29 vor einer unmittelbaren Bestrahlung möglichst geschützt wird. Zu diesem Zweck erfolgen verschiedene Einzelapplikationen, in Form von durch verschiedene Blendenöffnungen 8 bestrahlte annähernd zylindrische Räume 28, die durch den Körper hindurchgehen. Zunächst darf dabei möglichst keiner dieser Teilräume 28 der Bestrahlung das kritische Gewebe 29 unmittelbar treffen. Beim übrigen umliegenden Gewebe sollen sich diese aus vielen Richtungen kommenden Einzelapplikationen möglichst nicht überschneiden, damit das umliegende Gewebe eine wesentlich geringere Strahlenbelastung erhält als das Behandlungsobjekt 4.

Bei der **Fig. 4** wurde in ganz vereinfachter Darstellung gezeigt, wie mittels Applikationen, die in der Zeichenebene verlaufen - die Achsen 11 der bestrahlten Räume 28 sind strichpunktiert dargestellt - und Applikationen, die senkrecht zur Zeichnung verlaufen - die Achsen 11 der bestrahlten Räume 28 sind durch Kreuze symbolisiert - eine Vervielfachung der Strahlendosis im Behandlungsobjekt 4 erzielt werden kann. Bei dieser vereinfachten Darstellung wäre natürlich die Intensität der Bestrahlung im Behandlungsobjekt 4 noch zu gering; es ist also erforderlich, diese Applikationen derart vorzunehmen, daß sich die Bestrahlungsintensität im Behandlungsobjekt 4 noch wesentlich erhöht. Dies wird dadurch erreicht, daß nicht nur, wie dargestellt, waagerecht und senkrecht zur Zeichenebene Strahlenapplikationen vorgenommen werden, sondern daß diese aus allen Raumwinkeln erfolgen, die möglich sind. Möglich bedeutet in diesem Sinne nicht geometrisch möglich, sondern mit der Einschränkung, daß kritisches Gewebe 29, wie beispielsweise das dargestellte, möglichst ausgespart werden muß. Unter diesen Bedingungen ist es möglich, daß das Behandlungsobjekt 4 eine so starke Strahlendosis erhält, daß beispielsweise das Tumorgewebe zerstört wird und dabei das umliegende Gewebe so wenig Strahlung erhält, daß es sich wieder regenerieren kann, beziehungsweise das kritische Gewebe außer wenigen unvermeidbaren Streustrahlen keine oder nur sehr geringe Bestrahlung erhält.

Man kann sich in Anbetracht der erforderlichen vielfachen Überlagerung von in allen Richtungen ausgerichteten Teilräumen 28, welche durch die vielen Einzelapplikationen bestrahlt werden, vorstellen, daß dies sehr komplexe Rechenoperationen voraussetzt. Könnte man dabei nicht von Vielecken 12 als Querschnitte dieser Räume 28 ausgehen, welche der Kreisform sehr nahe kommen und daher rechnerisch als Kreis, beziehungsweise den Raum als Zylinder, betrachtet werden können, so würde diese Vorgehensweise in einem unwirtschaftlichen Maß komplex werden. Eine derartige Komplexität resultiert daraus, daß eine Berücksichtigung der Vielecke vorgenommen werden müßte, wenn es sich beim Querschnitt der Strahlen 2' beispielsweise um vier-oder sechseckige handeln würde, zumal die Ausrichtung der Vielecke bei jeder Applikation anders gelagert ist und daher rechnerisch sowohl die Vielecke als auch ihre jeweilige Ausrichtung berücksichtigt werden müßten. Dies wäre bei einer Vielzahl von Einstrahlrichtungen mit einer angemessenen Rechnerkapazität und einer angemessenen Rechenzeit nicht mehr wirtschaftlich bewältigbar. Darum schafft die Erfindung durch die Anordnung von mindestens zwei gleichachsigen Irisblenden die Vorraussetzung dafür, daß die Strahlenquerschnitte der Strahlen 2' derartige Vielecke 12 sind, daß die Räume 28 der Einzelapplikationen als Zylinder behandelt werden können, ohne eine Ungenauigkeit damit in Kauf zu nehmen, die der Bildung eines exakten Strahlenprofils entgegenstehen würde. Auf der anderen Seite ist es durch die Erfindung möglich, Irisblenden 5, 6 einzusetzen, deren Mechanik wesentlich einfacher und beherrschbarer ist, wie dies bei einer Irisblende der Fall wäre, welche aufgrund ihrer Blendenblattzahl sofort das Vieleck 12 in der gewünschten Form bildet.

Dabei hat die Anordnung der erfindungsgemäßen Irisblenden gleichzeitig den weiteren Vorteil, daß durch den Winkelversatz vermieden wird, daß die Leckstrahlen der ersten Irisblende 5, welche durch die Aneinandergrenzungen der Blendenblätter 9, 9', 9", 9"', 9"", 9""', also an den aneinandergrenzenden Seitenflächen 10 hindurchgehen, durch die weitere Irisblende 6 voll aufgefangen werden. Außerdem ist es auf einfache Weise möglich, beim Einsatz zweier derartiger Irisblenden 5, 6 die jeweiligen Blendenöffnungen 8' und 8" derart einzustellen, daß innerhalb des gesamten Stellbereichs der Divergenz der Strahlen 2', bei deren Begrenzung Rechnung getragen wird. Dies ist besonders wichtig, wenn weite Öffnungen 8, 8" der Irisblenden erfolgen sollen, um - wie dies der Fig. 4 zu entnehmen ist - durch Strahlenbündel 2' großen Durchmessers in einer Applikation große Bereiche des Behandlungsobjektes 4 zu erfassen, was die Behandlungszeit wesentlich verkürzt.

Gerade bei solchen Strahtenbündeln 2' großen Durchmessers würden bei gleichen Blendenöffnungen 8', 8" auch größere Halbschatten entstehen, welche durch die unterschiedlichen, der Divergenz der Strahlen 2' angepaßten Blendenöffnungen 8', 8" verringert werden können, wie dies aus Fig. 1 ersichtlich ist. Dieser Effekt kann auch noch erhöht werden, wenn man mehr als zwei Irisblenden 5, 6 in den Strahlengang bringt, da sich die Verringerung des Halbschattens an der Zahl der Irisblenden 5, 6 entsprechend deren Anzahl auf die Hälfte, ⅓ oder ¼ reduziert.

Die Fig. 4 zeigt letztlich nur eine starke Vereinfachung zur Erläuterung des Prinzips, wie sich aus vielen verschieden großen Strahlenbündeln 2' eine Erhöhung der Strahlendosis auch in einem völlig unregelmäßigen Raum eines Behandlungsobjektes 4 erzielen läßt. Wenn sich aus allen Richtungen kommenden Strahlenbündel dort entsprechend überlagern, kann im Verhältnis zur Darstellung noch eine wesentliche Vervielfachung der Strahlendosis im Behandlungsobjekt 4 erzielt werden.

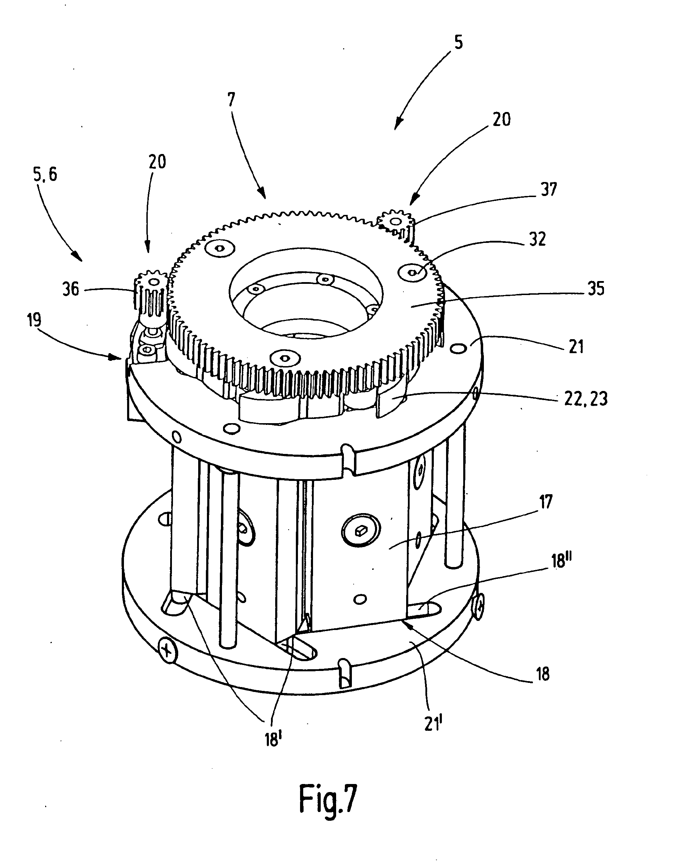

**Fig. 5** zeigt ein Ausführungsbeispiel einer erfindungsgemäßen Bestrahlungsvorrichtung in perspektivischer Darstellung. Dabei sind zwei Irisblenden 5 und 6 gleichachsig übereinander angeordnet, um Blendenöffnungen 8' und 8" der oben genannten Art zu erzeugen. Die Blendenblätter 9, 9', 9", 9"', 9"" und 9""' der jeweiligen Irisblende 5 und 6 sind derart aufgebaut, wie dies noch zu Fig. 9 erläutert wird. Sie bestehen aus innenliegenden Abschirmelementen 16, welche durch die obere Öffnung sichtbar sind, und äußeren Lagerelementen 17, die am Umfang zu sehen sind. Die Betätigung der Blendenblätter 9, 9', 9", 9"', 9"", 9""' der beiden Irisblenden 5 und 6 erfolgt dadurch, daß bolzenförmige Mitnehmer 24, vorzugsweise als Rollen ausgebildet, von den Mitnehmerkurven 23 einer Kurvenscheibe 22 betätigt werden. Die Mitnehmerkurven 23 sind derart sternförmig angeordnet, daß dem bolzenförmigen Mitnehmer 24 eines jeden Blendenblatts 9, 9', 9", 9"', 9"", 9""' eine solche Mitnehmerkurve 23 zugeordnet ist. Die bolzenförmigen Mitnehmer 24 greifen durch Durchgriffsschlitze 34 einer Führungsplatte 21 hindurch, um die Stellbewegung den Blendenblättern 9, 9', 9", 9"', 9"", 9""' zu übermitteln. An der Unterseite einer jeden Irisblende 5 und 6 ist eine weitere Führungsplatte 21' angeordnet, in der die Blendenblätter 9, 9', 9", 9"', 9"", 9""' ebenfalls in Linearführungen 18 geführt sind, um eine stabile Führung zu erzielen, insbesondere um ein Kippen zu vermeiden. Die Linearfiihrungen 18 bestehen aus einem Führungsteil 18' an den Blendenblättern 9, 9', 9", 9"', 9"", 9""' und einem mit diesem Führungsteil 18' zusammenwirkenden Führungsteil 18" an der Führungsplatte 21 sowie entsprechend an der weiteren Führungsplatte 21' (siehe Fig. 7 und 9).

An der Oberseite der Kurvenscheibe 22 befinden sich Befestigungen 32 für einen Antrieb, beispielsweise für ein Antriebszahnrad 35 (siehe Fig. 7). Dabei ist es möglich, der zweiten Irisblende 6 an der Unterseite ebenfalls ein Antriebszahnrad 35 zuzuordnen, oder es kann vorgesehen sein, daß sich zwischen den beiden Irisblenden 5, 6 eine Stellmechanik befindet, welche dafür sorgt, daß die Stellbewegung der einen Irisblende 5 auf die andere Irisblende 6 übertragen wird. Dazu können an der Unterseite der Blendenblätter 9, 9', 9", 9"', 9'"', 9""' der Irisblende 5 Kraftübemagungsglieder vorgesehen sein, welche die Stellbewegung übertragen. Zweckmäßigerweise sollte dann eine Übersetzung vorgesehen sein, damit der Divergenz der Strahlen 2 entsprechende Blendenöffnungen 8' und 8" durch die beiden Irisblenden 5 und 6 erzeugbar sind. Beispielsweise könnten Stifte von den Blendenblättern 9, 9', 9", 9"', 9"", 9"'" der einen Irisblende 5 in eine Kurvenscheibe 22 für die zweite Irisblende 6 eingreifen, und dieser Eingriff durch schräg zur Bewegungsrichtung der Stifte verlaufende Nuten bewirkt werden, die durch ihre Schrägstellung die jeweils erforderliche Übersetzung bewirken.

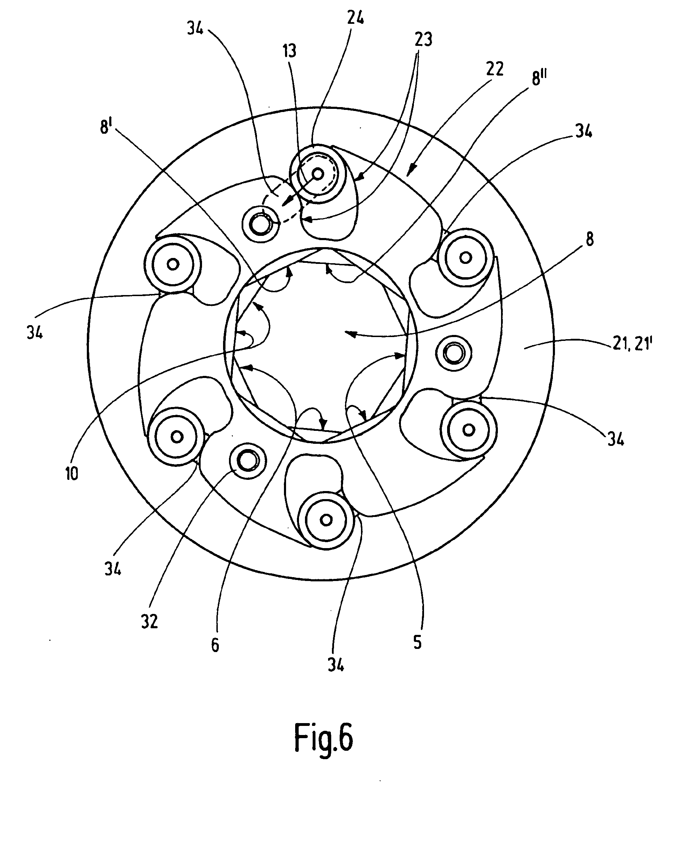

**Fig. 6** zeigt eine Draufsicht auf den Kollimator 1 gemäß Fig. 5. Dabei wird sichtbar, wie die Mitnehmerkurven 23 der Kurvenscheibe 22 auf die Rollen 24 wirken, damit diese und damit die mit ihnen verbundenen Blendenblätter 9, 9', 9", 9"', 9"", 9""' in Richtung der durch den Pfeil 13 für ein Blendenblatt angezeigten Stellbewegung bewegt werden können. Die Stellbewegungen 13 der Blendenblätter 9, 9', 9", 9'", 9"", 9'"" verlaufen wie in Fig. 3c eingezeichnet. Diese Stellbewegungen 13 werden den Blendenblättern 9, 9', 9", 9"', 9"", 9""' dadurch vermittelt, daß die bolzenförmigen Mitnehmer 24 durch die Durchgriffschlitze 34 der Führungsplatte 21 oder auch einer weiteren Führungsplatte 21' hindurchgreifen. Um zu zeigen, wie die Durchgriffschlitze 34 verlaufen, ist der oberste Durchgriffschlitz 34 in gestrichelter Linie eingezeichnet. In entsprechender Weise auf die Mitnehmerkurven 23 abgestimmt verlaufen alle sechs Durchgriffschlitze 34.

Im Bereich der Blendenöffnung 8 ist sowohl die Blendenöffnung 8' der ersten Irisblende 5, als auch die Blendenöffnung 8" der Irisblende 6 sichtbar, wobei hier die Teile der Seitenflächen 10 der Blendenblätter 9, 9', 9", 9"', 9"", 9""' zu sehen sind, welche die Blendenöffnung 8' bzw. 8" begrenzen. An der Oberseite sind die Befestigungen 32 für ein Antriebszahnrad 35 zu sehen.

**Fig. 7** zeigt eine Irisblende 5 mit Antrieb 19 und Getriebe 20. Die Darstellung entspricht der Fig. 5, wobei hier jedoch nur eine der beiden Irisblenden 5, 6 gezeichnet ist. Bei dieser Darstellung ist auf der Kurvenscheibe 22 das Antriebszahnrad 35 mittels Befestigungen 32 angebracht, um die Stellbewegung zu bewirken. Vom Antrieb 19 ausgehend greift ein erstes Ritzel 36 des Getriebes 20 in das Antriebszahnrad 35 ein und ein zweites Ritzel 37 ist angeordnet, welches mittels einer Welle den Antrieb auf ein weiteres Antriebszahnrad 35 übermittelt, welches der (hier nicht dargestellten) zweiten Irisblende 6 zugeordnet ist. Bei einer solchen Ausgestaltung eines Getriebes 20 kann die Übersetzung für jede der Irisblenden 5 oder 6 leicht dadurch variiert werden, daß die Ritzel 36 oder 37 und die Antriebszahnräder 35 durch solche mit anderer Zähnezahl ersetzt werden. Auf diese Weise läßt sich ein Kollimator 1 leicht an eine andere Strahlendivergenz eines anderen Bestrahlungsgeräts anpassen.

In **Fig. 7** sind noch die Linearführungen 18 für die Lagerelemente 17 der Blendenblätter 9, 9', 9", 9"', 9"", 9'"" besser sichtbar, da die gezeichnete Irisblende 5 sich nicht in der größten Öffnungsstellung befindet, wie dies bei der Darstellung der Fig. 5 der Fall war. Auf diese Weise ist zu sehen, wie sich die Führungsteile 18' der Blendenblätter 9, 9', 9", 9"', 9"", 9""' in den Führungsteilen 18" der Führungsplatte 21' bewegen. Zu sehen ist dies allerdings nur bei der weiteren Führungsplatte 21', bei der oberen Führungsplatte 21 sieht dies entsprechend aus.

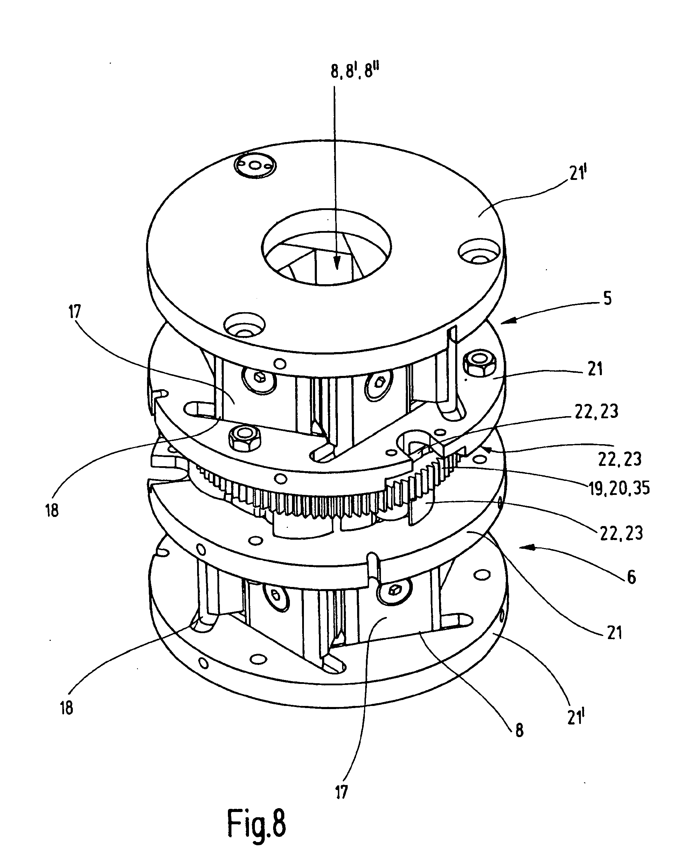

**Fig. 8** zeigt ein weiteres Ausführungsbeispiel des erfindungsgemäßen Kollimators 1 mit einem Antrieb 19, der zwischen zwei Irisblenden 5, 6 angeordnet ist. Hier befindet sich ein einziges Antriebszahnrad 35 zwischen zwei Kurvenscheiben 22, die jeweils einer der Irisblenden 5 bzw. 6 zugeordnet sind. Auch hier greift über einen Antrieb 19 ein (hier nicht dargestelltes) Ritzel in das Zahnrad 35 ein, um ein Getriebe 20 zu bilden, welches die Stellbewegung vollzieht. Da in diesem Fall das Antriebszahnrad 35 für beide Irisblenden 5 und 6 dieselben Stellwege vollzieht, müssen die Stellwege 14, die ja für die verschiedenen Irisblenden 5 und 6 entsprechend der Divergenz der Strahlen 2 unterschiedlich sein sollen, dadurch bewirkt werden, daß die Mitnehmerkurven 23 der Kurvenscheiben 22 für jede Irisblende 5 bzw. 6 und/oder die Schräge der Durchgriffschlitze 34 unterschiedlich ausgebildet sind. Unterschiedlich in dem Sinn, daß bei identischen Stellwinkeln für die Irisblenden 5 oder 6 auch unterschiedliche Stellwege den bolzenförmigen Mitnehmern 24 vermittelt werden. Dies kann beispielsweise dadurch geschehen, daß unterschiedlich große Kurvenscheiben 22 die Mitnehmer 24 der Irisblenden 5, 6 an verschiedenen Hebeln unterschiedlich weit bewegen und somit ein der Strahlendivergenz entsprechendes Übersetzungsverhältnis zur Bewirkung der Blendenöffnungen 8, 8' der Irisblenden 5, 6 ermöglichen. Die übrigen Bezugszeichen zeigen dieselben Teile wie bereits oben beschrieben.

**Fig. 9** zeigt eine besonders vorteilhafte Ausgestaltung eines Blendenblatts 9, 9', 9", 9"', 9"" oder 9""' für den erfindungsgemäßen Kollimator 5 oder 6. Das Blendenblatt 9, 9', 9", 9"', 9"" oder 9""' weist ein Abschirmelement 16 auf, das mittels einer Lagerung 30 in einem Lagerelement 17 gelagert ist. Diese Lagerung 30 mit einer Führung 40 (nur am Abschirmelement 16 sichtbar, eine zur sichtbaren Lagerung 30 komplementäre Ausnehmung befindet sich am Lagerelement 17) ist unter Einschluß von Federn 26 und durch Befestigung mit einer Schraube 31 derart ausgebildet, daß ein Federweg in Richtung des Doppelpfeils 38 entsteht, durch welchen sich das Abschirmelement 16 federnd auf dem Lagerelement 17 abstützt. Auf diese Weise wird erreicht, daß die Abschirmelemente 16 aller Blendenblätter 9, 9', 9", 9"', 9"", 9""' mit ihren Seitenflächen 10 immer plan aufeinanderliegen. Dabei ist die Lagerung 30 auf der Rückfläche 39 des Abschirmelements 16 angeordnet. (Damit ersichtlich ist, wo sich diese befindet, ist diese Rückfläche 39 bei dem Blendenblatt 9 der Fig. 3c eingezeichnet.) Aus der Zusammenschau der Figuren 9 und 3c ergibt sich, daß die auf das Blendenblatt 9 wirkende Federkraft in Richtung der Winkelhalbierenden 25 wirkt, die sich in zwei gleiche Kraftkomponenten aufteilt, die in Richtung der Blendenblätter 9' und 9""' gehen. Entsprechend ist dies für jedes Blendenblatt 9, 9', 9", 9"', 9"", 9""'. Die Größe der Abschirmelemente 16 ist auf den Strahl 2 derart abgestimmt, daß auch bei geschlossener Irisblende 5 oder 6 (siehe Fig. 3a) der gesamte Strahl 2 abgeschirmt wird. Der Strahl 2 ist mittels des Vorkollimators 27 entsprechend limitiert (siehe Fig. 1).

Das Lagerelement 17 der Blendenblätter 9, 9', 9", 9"', 9"", 9""' trägt zwei Führungsteile 18', welche mit zwei Führungsteilen 18" der Führungsplatte 21 und der weiteren Führungsplatte 21' zusammenwirken. Mindestens eines dieser Führungsteile 18' weist ein Befestigungsgewinde 33 für einen Mitnehmer 24 auf, der durch einen Durchgriffschlitz 34 einer Führungsplatte 21 oder 21' hindurchgreifend über die Kurvenscheibe 22 eine Verbindung zum Antrieb 19 herstellt.

Auf die besondere Bedeutung der Ausgestaltung der Blendenblätter 9, 9', 9", 9"', 9"", 9'"" mit Abschirmelement 16 und Lagerelement 17 wurde oben hingewiesen, nämlich daß die plane Anlage aller Seitenflächen 10 auf diese Weise trotz Toleranzabweichungen immer erreicht werden kann. Selbstverständlich handelt es sich hierbei lediglich um eine Ausführungsform, auch andere Ausgestaltungen wären denkbar, wie beispielsweise die Anordnung eines elastischen Werkstoffs zwischen dem Abschirmelement 16 und dem Lagerelement 17.

Die Darstellungen zeigen lediglich eine von vielen möglichen Ausführungsformen der Erfindung, selbstverständlich könnten Antriebe und die Ausgestaltung der Blendenblätter 9, 9', 9", 9"', 9"", 9""' auch in anderen Formen ausgeführt werden. Auch andere Linearführungen wären denkbar. Als Kurvenscheiben 22 wären ebenfalls andere Gestaltungen möglich, wie beispielsweise die Führung von bolzenförmigen Mitnehmern 24 in spiralartigen Kurven. Wie bereits oben angemerkt, können natürlich auch mehr als zwei Irisblenden 5, 6 angeordnet werden, und es ist auch möglich, Irisblenden mit anderen Blendenblattzahlen zu verwenden. Das Wesen der Erfindung besteht letztlich darin, daß mindestens zwei Irisblenden 5, 6 angeordnet werden, um trotz einer beschränkten Anzahl von Blendenblättern 9, 9', 9", 9"', 9"", 9""' Vielecke 12 zu erzielen, welche einer Kreisform sehr nahekommen, um die beschriebene exakte Strahlenapplikation bei relativ kurzer Behandlungsdauer zu ermöglichen und gleichzeitig die Leckstrahlung zwischen den Blendenblättern 9, 9', 9", 9"', 9"", 9'"" zu minimieren.

### Bezugszeichenliste

- 1: Kollimator
- 2: Strahlen
- 2': Strahlen durch den Kollimator begrenzt (im wesentlichen runder Strahlenquerschnitt)
- 3: Strahlungsquelle
- 4: Behandlungsobjekt
- 5: Irisblende
- 6: weitere Irisblende
- 7: Stellmittel
- 8, 8', 8": Blendenöffnung
- 8': der ersten Irisblende 5
- 8": der zweiten Irisblende 6
- 9 9',9",9"' 9"", 9'"": Blendenblätter bei einer sechsblättrigen Irisblende
- 10: Seitenflächen
- 11: Achse
- 12: Vieleck (durch Kollimator gebildet)
- 13: Pfeile: geradlinige Stellbewegung
- 14: Stellwege
- 15: Bestrahlungskopf
- 16: Abschirmelement der Blendenblätter
- 17: Lagerelement der Blendenblätter
- 18: Linearführungen
- 18': Führungsteil an den Blendenblättern
- 18": Führungsteil an der Führungsplatte
- 19: Antrieb
- 20: Getriebe
- 21: Führungsplatte
- 21': weitere Führungsplatte
- 22: Kurvenscheibe
- 23: Mitnehmerkurven, sternförmig angeordnet
- 24: bolzenförmiger Mitnehmer, vorzugsweise Rollen
- 25: Winkelhalbierende zwischen den Seitenflächen 10
- 26: Federn zwischen Abschirmteil und Lagerteil
- 27: Vorkollimator
- 28: bestrahlter Raum, Teilraum
- 29: kritisches Gewebe (z. B. Nerv)
- 30: Lagerung
- 31: Schraube
- 32: Befestigung für Antriebszahnrad
- 33: Befestigungsgewinde für Mitnehmer 24
- 34: Durchgriffsschlitz für Mitnehmer 24
- 35: Antriebszahnrad
- 36: erstes Ritzel
- 37: zweites Ritzel
- 38: Doppelpfeil: Richtung des Federwegs
- 39: Rückfläche eines Abschirmelements
- 40: Führung

- α: Versatz der Irisblenden in Drehrichtung um die Achse 11

## Patentansprüche

1. Kollimator (1) zum Begrenzen energiereicher Strahlen (2), die von einer im wesentlichen punktförmigen Strahlungsquelle (3) ausgehend auf ein Behandlungsobjekt (4) gerichtet sind und der Strahlenbehandlung insbesondere der stereotaktischen Konformationsbestrahlung von Tumoren dient, wobei der Kollimator (1) eine als Irisblende (5) mit Stellmitteln (7) ausgebildete Strahlbegrenzung zur Erzielung einer variablen Blendenöffnung (8, 8') aufweist,
**dadurch gekennzeichnet,**
**daß** sich mindestens eine weitere Irisblende (6) gleichachsig im Strahlengang befindet, wobei die Irisblenden (5, 6) derart angeordnet sind, daß die jeweiligen Blendenblätter (9, 9', 9", 9"', 9"", 9""') der Blenden (5, 6) derart gegeneinander in Drehrichtung um ihre Achse (11) versetzt sind, daß der durch den Kollimator (1) begrenzte Strahl (2') den Querschnitt eines Vielecks (12) hat, dessen Eckenzahl der Zahl der Blendenblätter aller Irisblenden (5, 6) entspricht, und daß die Blendenblätter (9, 9', 9", 9"', 9"", 9""') gleiche Winkel einschließende, aneinanderliegende Seitenflächen (10) aufweisen, wobei die Stellbewegungen (13) der Blendenblätter (9, 9', 9", 9"', 9"", 9""') auf geradlinige Weise senkrecht zur Winkelhalbierenden (25) der mit den angrenzenden Blendenblättern in Eingriff befindlichen Seitenflächen (10) verlaufen.

2. Kollimator nach Anspruch 1,
**dadurch gekennzeichnet,**
**daß** durch die gleiche Blendenblattzahl der mindestens zwei Irisblenden (5, 6), durch einen entsprechenden Winkelversatz (α) derselben in Drehrichtung um die Achse (11) und durch die Ausbildung der Stellmittel (7) der Irisblenden (5, 6) mit gleichen Stellwegen (14, 14') für die Blendenblätter (9, 9', 9", 9"', 9"", 9""') der jeweiligen Irisblende (5, 6) ein Querschnitt eines gleichseitigen Vielecks (12) der Strahlen (2') im gesamten Einstellbereich gebildet wird.

3. Kollimator nach Anspruch 1 oder 2,
**dadurch gekennzeichnet,**
**daß** Kraftbeaufschlagungen vorgesehen sind, die die Blendenblätter (9, 9', 9", 9"', 9"", 9""') gegeneinanderdrücken.

4. Kollimator nach Anspruch 1, 2 oder 3,
**dadurch gekennzeichnet,**
**daß** die Blendenblätter (9, 9', 9", 9"', 9"", 9""') aus einem Abschirmelement (16) aus Abschirmmaterial und einem Lagerelement (17) bestehen, wobei das Lagerelement (17) ein Führungsteil (18') einer Linearführung (18) für die Stellbewegung (13) aufweist und das Abschirmelement (16) auf dem Lagerelement (17) derart federgelagert ist, daß es gleichmäßig in Richtung der angrenzenden Blendenblätter gedrückt wird.

5. Bestrahlungsvorrichtung mit einem Kollimator (1) gemäß Anspruch 1, wobei eine Einrichtung vorgesehen ist, durch welche die durch den Kollimator (1) begrenzten Strahlen (2') allseitig auf das Behandlungsobjekt (4) richtbar sind, und wobei mittels einer Steuerung die Parameter Bestrahlungsrichtung, Bestrahlungsfläche, Bestrahlungsintensität und -zeit derart steuerbar sind, daß ein räumliches Dosierungsprofil der Strahlenapplikation erzielbar ist, und daß die Steuerung derart ausgebildet ist, daß sie die Bestrahlung eines unregelmäßigen Raums durch Überlagern und Aneinanderfügen vieler bestrahlter Räume (28) bewirken kann.

6. Bestrahlungsvorrichtung nach Anspruch 5,
**dadurch gekennzeichnet,**
**daß** die Stellmittel (7) der Irisblenden (5, 6) derart ausgebildet sind, daß die jeweiligen Blendenöffnungen (8, 8', 8") innerhalb des gesamten Stellbereichs der Divergenz der Strahlen (2') bei deren Begrenzung Rechnung tragen

7. Bestrahlungsvorrichtung nach Anspruch 5 oder 6,
**dadurch gekennzeichnet,**
**daß** durch die gleiche Blendenblattzahl der mindestens zwei Irisblenden (5, 6), durch einen entsprechenden Winkelversatz (α) derselben in Drehrichtung um die Achse (11) und durch die Ausbildung der Stellmittel (7) der Irisblenden (5, 6) mit gleichen Stellwegen (14, 14') für die Blendenblätter (9, 9', 9", 9"', 9"", 9'"") der jeweiligen Irisblende (5, 6) ein Querschnitt eines gleichseitigen Vielecks (12) der Strahlen (2') im gesamten Einstellbereich gebildet wird.

8. Bestrahlungsvorrichtung nach Anspruch 5, 6 oder 7,
**dadurch gekennzeichnet,**
**daß** die Steuerung derart ausgebildet ist, daß aufgrund von verschiedenen Blendenöffnungen (8, 8', 8"), Positionierungen von Strahlungsquelle (3) und Kollimator (1) im Raum und der Ausrichtung in bestimmten Raumwinkeln Einzelapplikationen gebildet werden, wobei durch eine Zusammenfügung einer Vielzahl dieser lediglich begrenzte Teilräume (28) des Behandlungsobjektes (4) erfassenden bestrahlten Räume (28) der Einzelapplikationen mit einem im wesentlichen runden Strahlquerschnitt (2') - also einer von der Form des Behandlungsobjekts (4) in der Regel abweichenden Gestalt - die in der Regel unregelmäßige Raumform des Behandlungsobjekts (4) nachgebildet werden kann, wobei durch mehrfache sowohl Nebeneinander-, als auch Übereinanderlagerung, einschließlich teilweiser Neben- und Übereinanderlagerung, dieser Teilräume (28) erfassenden Einzelapplikationen im Raum des Behandlungsobjektes (4) dieses mit relativ exakten Grenzen in vorgegebener Intensität wesentlich höher bestrahlt werden kann als das umliegende Gewebe.

9. Bestrahlungsvorrichtung nach Anspruch 8,
**dadurch gekennzeichnet,**
**daß** die Einzelapplikationen jeweils in einen gewissen Zeitraum mit unveränderten Parametern erfolgen.

10. Bestrahlungsvorrichtung nach einem der Ansprüche 5 bis 9,
**dadurch gekennzeichnet,**
**daß** Kraftbeaufschlagungen vorgesehen sind, die die Seitenflächen (10) der Blendenblätter (9, 9', 9", 9'", 9"", 9'"") gegeneinanderdrücken.

11. Bestrahlungsvorrichtung nach Anspruch 10,
**dadurch gekennzeichnet,**
**daß** die Blendenblätter (9, 9', 9", 9"', 9"", 9""') aus einem Abschirmelement (16) aus Abschirmmaterial und einem Lagerelement (17) bestehen, wobei das Lagerelement (17) ein Führungsteil (18') einer Linearführung (18) für die Stellbewegung (13) aufweist und das Abschirmelement (16) auf dem Lagerelement (17) derart federgelagert ist, daß es gleichmäßig in Richtung der angrenzenden Blendenblätter gedrückt wird.

12. Bestrahlungsvorrichtung nach einem der Ansprüche 5 bis 11,
**dadurch gekennzeichnet,**
**daß** ein Bestrahlungskopf (15), der die Strahlungsquelle (3) und den Kollimator (1) enthält, auf einer Gantry angeordnet ist.

13. Bestrahlungsvorrichtung nach einem der Ansprüche 5 bis 11,
**dadurch gekennzeichnet,**
**daß** ein Bestrahlungskopf (15), der die Strahlungsquelle (3) und den Kollimator (1) enthält, auf einem Roboterarm angeordnet ist, der derart ausgebildet ist, daß er den Bestrahlungskopf (15) in beliebige Raumpositionen und beliebige Raumwinkelausrichtungen bringen kann.

14. Bestrahlungsvorrichtung nach einem der Ansprüche 5 bis 13,
**dadurch gekennzeichnet,**
**daß** der Kollimator (1) für Schwenkbewegungen in einem begrenzten Raumwinkelbereich auf einer kugeloberflächenförmigen Bahn allseitig verschiebbar gelagert ist, wobei die Achse (11) immer auf die Strahlungsquelle (3) ausgerichtet ist.

15. Bestrahlungsvorrichtung nach einem der Ansprüche 5 bis 14,
**dadurch gekennzeichnet,**
**daß** jede Irisblende (5,6) über einen Antrieb (19) verfügt.

16. Bestrahlungsvorrichtung nach Anspruch 15,
**dadurch gekennzeichnet,**
**daß** die Steuerung die jeweilige Blendenöffnung (8, 8', 8") entsprechend der Divergenz der Strahlen (2') steuert.

17. Bestrahlungsvorrichtung nach einem der Ansprüche 5 bis 14,
**dadurch gekennzeichnet,**
**daß** die Irisblenden (5, 6) mittels eines einzigen Antriebs (19) betätigbar sind.

18. Bestrahlungsvorrichtung nach Anspruch 17,
**dadurch gekennzeichnet,**
**daß** eine Stellmechanik die jeweilige Blendenöffnung (8, 8', 8") entsprechend der Divergenz der Strahlen (2') bewirkt.

19. Bestrahlungsvorrichtung nach einem der Ansprüche 5 bis 18,
**dadurch gekennzeichnet,**
**daß** die Blendenblätter (9, 9', 9", 9"', 9"", 9'"") einer jeden Irisblende (5, 6) in mindestens einer einen Führungsteil (18") der Linearführungen (18) enthaltenden Führungsplatte (21) geführt sind.

20. Bestrahlungsvorrichtung nach Anspruch 18,
**dadurch gekennzeichnet,**
**daß** eine Kurvenscheibe (22) mit den Blendenblättern (9, 9', 9", 9"', 9"", 9""') in Wirkverbindung steht und daß der Antrieb (19) die Stellbewegungen (13) der Blendenblätter (9, 9', 9", 9'", 9"", 9'"") über eine relative Drehbewegung zwischen Führungsplatte (21) und Kurvenscheibe (22) bewirkt.

21. Bestrahlungsvorrichtung nach Anspruch 18 oder 19,
**dadurch gekennzeichnet,**
**daß** die Blendenblätter (9, 9', 9", 9"', 9"", 9""') einer jeden Irisblende (5, 6) auch an der gegenüberliegenden Seite mittels einer weiteren Führungsplatte (21') geführt sind.

22. Bestrahlungsvorrichtung nach Anspruch 16 bis 20,
**dadurch gekennzeichnet,**
**daß** der Antrieb (19) zwischen zwei Irisblenden (5, 6) eingreift, um von dort beide Irisblenden (5, 6) zu betätigen.

23. Bestrahlungsvorrichtung nach Anspruch 16 bis 21,
**dadurch gekennzeichnet,**
**daß** der Antrieb (19) einer Irisblende (5) zugeordnet ist und über Mitnehmer der Antrieb der weiteren Irisblende (6) bewirkt wird, wobei die Kraftübertragungselemente für die Bewirkung von der Divergenz der Strahlen (2') entsprechenden Blendenöffnungen (8, 8', 8") ausgestaltet sind.

24. Bestrahlungsvorrichtung nach Anspruch 16 bis 22,
**dadurch gekennzeichnet,**
**daß** die Stellmittel (7) für mehrere Krafteinleitungen des Antriebs (19) ausgebildet sind.

25. Bestrahlungsvorrichtung nach Anspruch 24,
**dadurch gekennzeichnet,**
**daß** mindestens einer Krafteinleitung ein Getriebe (20) zugeordnet ist, durch das ein der Divergenz der Strahlen (2') entsprechendes Verhältnis der Blendenöffnungen (8, 8', 8") bewirkt wird.

26. Bestrahlungsvorrichtung nach Anspruch 25,
**dadurch gekennzeichnet,**
**daß** das mindestens eine Getriebe (20) mindestens ein austausch- oder verstellbares Übertragungsglied aufweist, um den Kollimator (1) auf verschiedene Strahldivergenzen einzustellen.

27. Bestrahlungsvorrichtung nach einem der Ansprüche 20 bis 26,
**dadurch gekennzeichnet,**
**daß** die Kurvenscheiben (22) sternförmig angeordnete Mitnehmerkurven (23) aufweisen, die auf bolzenförmige Mitnehmer (24) der Blendenblätter (9, 9', 9", 9"',9"",9'""') wirken.

28. Bestrahlungsvorrichtung nach Anspruch 27,
**dadurch gekennzeichnet,**
**daß** die bolzenförmigen Mitnehmer (24) Rollen sind.

29. Bestrahlungsvorrichtung nach einem der Ansprüche 5 bis 28,
**dadurch gekennzeichnet,**
**daß** zwei sechsblättrige, um 30° in Drehrichtung versetzte Irisblenden (5,6) angeordnet sind.

## Claims

1. A collimator (1) for delimiting a high-energy radiation beam (2) emanating from a substantially punctiform radiation source (3) and directed onto a therapy object (4), the radiation treatment being particularly suitable for the stereotactic conformation irradiation of tumors, which collimator (1) has beam limiting means in the form of an iris diaphragm (5) equipped with adjusting means (7) for varying the aperture (8, 8'),
**characterized in that**
at least one further iris diaphragm (6) is disposed coaxially in the optical path such that said iris diaphragms (5, 6) are arranged with the respective aperture leaves (9, 9', 9", 9"', 9"", 9""') of said diaphragms (5, 6) rotationally offset from each other about their axis (11) such that the beam (2') that is delimited by said collimator (1) has in cross-section the shape of a polygon (12), the number of corners of which is equal to the number of aperture leaves in all of said iris diaphragms (5, 6) and that said aperture leaves (9, 9', 9", 9"', 9"", 9""') have abutting lateral surfaces (10) all enclosing the same angle, wherein the adjustment movements (13) of said aperture leaves (9, 9', 9", 9"', 9"", 9""') are carried out along a straight line at right angles to the bisector (25) of the lateral surfaces (10) abutting adjacent aperture leaves.

2. The collimator as defined in claim 1,
**characterized in that**
said beam (2') is imparted with a cross-section having the shape of an equilateral polygon (12) over the entire adjustment range **in that** the at least two iris diaphragms (5, 6) each have the same number of aperture leaves, that an appropriate angular offset (a) thereof is set in the direction of rotation about said axis (11), and that said adjusting means (7) for said iris diaphragms (5, 6) are configured so as to effect the same adjustment displacements (14, 14') of said aperture leaves (9, 9", 9", 9"', 9"", 9""') in each iris diaphragm (5, 6).

3. The collimator as defined in claim 1 or claim 2,
**characterized in that**
force applying means are provided that press said aperture leaves (9, 9', 9", 9"', 9"", 9""') together.

4. The collimator as defined in claim 1, claim 2, or claim 3,
**characterized in that**
said aperture leaves (9, 9', 9", 9"', 9"", 9""') consist of a shielding element (16) of shielding material and a bearing element (17), which bearing element (17) has a guide member (18') of a linear guide (18) for effecting adjustment (13), and the shielding element (16) on the bearing element (17) is spring loaded in such a manner that it is pressed uniformly toward adjacent aperture leaves.

5. A radiation treatment device comprising a collimator (1) as defined in claim 1, wherein a system is provided, by means of which the radiation beam (2') delimited by said collimator (1) can be moved in all directions so as to aim at the therapy object (4), and by means of control means the parameters direction of irradiation, irradiation area, and intensity and duration of irradiation can be controlled in such a manner that a spatial dosage profile of the radiation application can be obtained, and that said control means are configured such that the irradiation of an irregular volume can be effected by superimposing and juxtaposing a plurality of irradiated volumes (28).

6. The radiation treatment device as defined in claim 5,
**characterized in that**
said adjusting means (7) for said iris diaphragms (5, 6) are configured in such a manner that the respective apertures (8, 8', 8") make allowance for the divergence of the radiation beam (2') for delimiting the same over the entire adjustment range.

7. The radiation treatment device as defined in claim 5 or claim 6,
**characterized in that**
a radiation beam (2') having, in cross-section, the shape of an equilateral polygon (12) is formed over the entire adjustment range **in that** the at least two iris diaphragms (5, 6) each have the same number of aperture leaves, that an appropriate angular offset (α) thereof is set in the direction of rotation about said axis (11), and that said adjusting means (7) for said iris diaphragms (5, 6) are configured so as to effect the same adjustment displacements (14, 14') of said aperture leaves (9, 9", 9", 9"', 9"", 9""') in each iris diaphragm (5, 6).

8. The radiation treatment device as defined in claim 5, claim 6, or claim 7,
**characterized in that**
said control means are such that individual applications are formed by varying the aperture (8, 8', 8"), the position of the radiation source (3) and collimator (1) in space and the orientation thereof in certain solid angles, and due to integration of a large number of these irradiated volumes (28) of individual applications covering only delimited sub-volumes (28) of the therapy object (4) and having a substantially circular radiation cross-section (2') - i.e. a shape usually deviating from the shape of the therapy object (4) -, it is possible to reconstruct the usually irregular three-dimensional shape of the therapy object (4), and by carrying out a number of individual applications involving juxtaposition and/or superposition, including partial juxtaposition and superposition, of these sub-volumes (28) of the spatial volume of the therapy object (4), the latter can be irradiated within relatively precise limits at a much higher specific intensity than the surrounding tissue.

9. The radiation treatment device as defined in claim 8,
**characterized in that**
said individual applications are each carried out within a predefined time frame with unchanged parameters.

10. The radiation treatment device as defined in any one of claims 5 to 9,
**characterized in that**
force applying means are provided that press the lateral surfaces (10) of said aperture leaves (9, 9', 9", 9"', 9"", 9""') together.

11. The radiation treatment device as defined in claim 10,
**characterized in that**
said aperture leaves (9, 9', 9", 9"', 9"", 9""') consist of a shielding element (16) of shielding material and a bearing element (17), which bearing element (17) has a guide member (18') of a linear guide (18) for effecting adjustment movements (13), and the shielding element (16) on the bearing element (17) is spring loaded in such a manner that it is pressed uniformly toward adjacent aperture leaves.

12. The radiation treatment device as defined in any one of claims 5 to 11,
**characterized in that**
a radiation head (15) containing said radiation source (3) and said collimator (1) is situated on a gantry.

13. The radiation treatment device as defined in any one of claims 5 to 11,
**characterized in that**
a radiation head (15) containing said radiation source (3) and said collimator (1) is disposed on a robot arm configured such that it can move said radiation head (15) to any desired position in space and in any desired solid angle orientation.

14. The radiation treatment device as defined in any one of claims 5 to 13,
**characterized in that**
said collimator (1) is mounted on a spherically shaped path for effecting swiveling movements in a restricted solid angle volume, which path is displaceable in all directions, while said axis (11) is always directed toward said radiation source (3).

15. The radiation treatment device as defined in any one of claims 5 to 14,
**characterized in that**
to each iris diaphragm (5.6) there is assigned a drive (19).

16. The radiation treatment device as defined in claim 15,
**characterized in that**
said control means control the respective aperture (8, 8', 8") according to the degree of divergence of said radiation beam (2').

17. The radiation treatment device as defined in any one of claims 5 to 14,
**characterized in that**
said iris diaphragms (5, 6) can be actuated by means of a single drive (19).

18. The radiation treatment device as defined in claim 17,
**characterized in that**
an adjusting mechanism sets the respective aperture (8, 8', 8") according to the degree of divergence of said radiation beam (2').

19. The radiation treatment device as defined in any one of claims 5 to 18,
**characterized in that**
said aperture leaves (9, 9', 9", 9"', 9"", 9""') of each iris diaphragm (5, 6) are guided in at least one guide plate (21) including a guide member (18") of said linear guides (18).

20. The radiation treatment device as defined in claim 18,
**characterized in that**
a disk cam (22) is actively connected to said aperture leaves (9, 9', 9", 9"', 9"", 9""'), and said drive (19) causes adjustment movements (13) of the aperture leaves (9, 9', 9", 9"', 9"", 9""') by a relative rotary movement between said guide plate (21) and said disk cam (22).

21. The radiation treatment device as defined in claim 18 or claim 19,
**characterized in that**
said aperture leaves (9, 9', 9", 9"', 9"", 9""') of each iris diaphragm (5, 6) are also guided on the opposite side by means of a further guide plate (21').

22. The radiation treatment device as defined in claim from 16 to 20,
**characterized in that**
said drive (19) engages between two iris diaphragms (5, 6), in order to actuate the two iris diaphragms (5, 6) from that position.

23. The radiation treatment device as defined any one of claims 16 to 21,
**characterized in that**
said drive (19) is assigned to one iris diaphragm (5) and driving of the other iris diaphragm (6) is effected via entrainers, whilst the power transmission elements for effecting such actuation are dependent on apertures (8, 8', 8") corresponding to the degree of divergence of the radiation beam (2').

24. The radiation treatment device as defined in claim from 16 to 22,
**characterized in that**
said adjusting means (7) are configured for a plurality of power flows from the drive (19).

25. The radiation treatment device as defined in claim 24,
**characterized in that**
a drive train (20) is assigned to at least one power flow to achieve a ratio of said apertures (8, 8', 8") corresponding to the degree of divergence of said radiation beam (2').

26. The radiation treatment device as defined in claim 25,
**characterized in that**
said at least one drive train (20) includes at least one replacement transfer element or variable transfer element to enable said collimator (1) to be adapted to various degrees of divergence of said radiation beam.

27. The radiation treatment device as defined in any one of claims 20 to 26,
**characterized in that**
said disk cams (22) comprise a star-like arrangement of driving cams (23), which act on pin-shaped entrainers (24) on said aperture leaves (9, 9', 9", 9"', 9"", 9""').

28. The radiation treatment device as defined in claim 27,
**characterized in that**
said pin-shaped entrainers (24) are rollers.

29. The radiation treatment device as defined in any one of claims 5 to 28,
**characterized in that**
two iris diaphragms (5.6) comprising six leaves mutually rotationally offset by 30° are present.

## Revendications

1. Collimateur (1) pour limiter des rayons riches en énergie (2) qui sont dirigés en partant d'une source de rayonnement sensiblement punctiforme (3) sur un objet de traitement (4) et sert au traitement par rayon en particulier à l'irradiation de conformation stéréotactique de tumeurs, le collimateur (1) présentant une limitation de rayon réalisée comme un diaphragme à iris (5) avec des moyens de réglage (7) pour obtenir une ouverture de diaphragme variable (8, 8'), **caractérisé en ce qu'**au moins un autre diaphragme à iris (6) se trouve sur le même axe dans la trajectoire du rayon, les diaphragmes à iris (5, 6) étant disposés de sorte que les lamelles de diaphragmes (9, 9', 9", 9"', 9'''', 9""') des diaphragmes (5, 6) sont décalées dans le sens de rotation autour de leur axe (11) de sorte que le rayon limité (2') par le collimateur (1) a une section transversale d'un polygone (12) dont le nombre d'angles correspond au nombre des lamelles de tous les diaphragmes (5, 6) et **en ce que** les lamelles de diaphragmes (9, 9', 9", 9"', 9"", 9""') présentent des surfaces latérales attenantes entourant des mêmes angles, les mouvements de réglage (13) des lamelles de diaphragmes (9, 9', 9", 9"', 9"", 9""') s'étendant de manière rectiligne perpendiculairement aux bissectrices (25) des surfaces latérales (10) se trouvant en engagement avec les lamelles de diaphragmes attenantes.

2. Collimateur selon la revendication 1,
**caractérisé en ce qu'**une section transversale d'un polygone équilatéral des rayons (2') est formée dans l'ensemble de la zone de réglage par le même nombre de lamelles d'au moins deux diaphragmes à iris (5, 6), par un déport angulaire correspondant (α) de ces derniers dans le sens de rotation autour de l'axe (11) et par la conception des moyens de réglage (7) des diaphragmes à iris (5, 6) avec des parcours de réglage identiques (14, 14') pour les lamelles de diaphragmes (9, 9', 9'', 9"', 9'''', 9""') des diaphragmes à iris (5, 6) respectifs.

3. Collimateur selon la revendication 1 ou 2, **caractérisé en ce qu'**il est prévu des applications de force pressant les lamelles de diaphragmes (9, 9', 9", 9"', 9"", 9""') les unes contre les autres.

4. Collimateur selon la revendication 1, 2 ou 3, **caractérisé en ce que** les lamelles de diaphragmes (9, 9', 9'', 9"', 9"", 9''''') se composent d'un élément formant écran (16) en matériau écran et d'un élément de palier(17), l'élément de palier (17) présentant une partie de guidage (18') d'un guide linéaire (18) pour le mouvement de réglage (13) et **en ce que** l'élément d'écran (16) est monté sur ressort sur l'élément de palier (17) de sorte qu'il est pressé de manière homogène en direction des lamelles de diaphragmes attenantes.

5. Dispositif d'irradiation avec un collimateur (1) selon la revendication 1, un dispositif étant prévu qui permet d'orienter des rayons (2'') limités par le collimateur (1) de part et d'autre de l'objet de traitement (4), et les paramètres tels que la direction d'irradiation, la surface d'irradiation, l'intensité et la durée d'irradiation étant commandable au moyen d'une commande de sorte qu'un profil de dosage spatial de l'application des rayons peut être obtenu, la commande étant réalisée de sorte qu'elle provoque l'irradiation d'un espace irrégulier par superposition et assemblement de nombreux espaces irradiés (28).

6. Dispositif d'irradiation selon la revendication 5, **caractérisé en ce que** le moyen de réglage (7) des diaphragmes à iris (5, 6) est réalisé de sorte que les ouvertures de diaphragme respectives (8, 8', 8" ) tiennent compte à l'intérieur de l'ensemble de la zone de réglage de la divergence des rayons (2') lors de la limitation.

7. Dispositif d'irradiation selon la revendication 5 ou 6 **caractérisé en ce qu'**une section transversale d'un polygone équilatéral (12) des rayons (2') est formé dans l'ensemble de la zone de réglage par le même nombre de lamelles d'au moins deux diaphragmes à iris (5, 6), par un déport angulaire correspondant (α) de ces derniers dans le sens de rotation autour de l'axe (11) et par la conception des moyens de réglage (7) des diaphragmes à iris (5, 6) avec des parcours de réglage identiques (14, 14') pour les lamelles de diaphragmes (9, 9', 9", 9"', 9"", 9""') des diaphragmes à iris (5, 6) respectifs.

8. Dispositif d'irradiation selon la revendication 5, 6 ou 7 **caractérisé en ce que** la commande est réalisée de sorte que sur la base d'ouvertures de diaphragmes différentes (8, 8', 8"), de positionnements de source de rayonnement (3) et du collimateur (1) dans l'espace et de l'orientation dans différents angles dans l'espace, il est réalisé des applications individuelles, par un assemblement d'un grand nombre de ces espaces irradiés (28), saisissant des espaces partiels seulement limités (28) de l'objet de traitement (4), des applications individuelles avec une section transversale de rayon relativement ronde (2') - donc une forme divergeant en règle générale de la forme de l'objet de traitement (4) -, la forme spatiale en général irrégulière de l'objet de traitement (4) peut être reproduite, par une superposition et une disposition les unes à côté des autres, y compris superposition et disposition partielles les unes à côté des autres de ces applications individuelles (28) saisissant des espaces partiels dans l'espace de l'objet de traitement (4) celui-ci peut être irradié de manière plus intense que le tissu environnant avec une intensité prescrite dans des limites relativement exactes.

9. Dispositif d'irradiation selon la revendication 8, **caractérisé en ce que** les applications individuelles s'effectuent respectivement dans une certaine période avec des paramètres modifiés.

10. Dispositif d'irradiation selon l'une des revendications 5 à 9, **caractérisé en ce que** il est prévu des applications de force pressant les lamelles de diaphragmes (9, 9', 9'', 9''', 9'''', 9''''') les unes contre les autres.

11. Dispositif d'irradiation selon la revendication 10, **caractérisé en ce que** les lamelles de diaphragmes (9, 9', 9'', 9''', 9'''', 9""') se composent d'un élément formant écran (16) en matériau écran et un élément de palier (17), l'élément de palier (17) présentant une partie de guidage (18') d'un guide linéaire (18) pour le mouvement de réglage (13) et **en ce que** l'élément d'écran (16) est monté sur ressort sur l'élément de palier (17) de sorte qu'il est pressé de manière homogène en direction des lamelles de diaphragmes attenantes.

12. Dispositif d'irradiation selon l'une des revendications 5 à 11, **caractérisé en ce qu'**une tête d'irradiation (15) contenant la source de rayonnement (3) et le collimateur (1) est disposée sur un portique.

13. Dispositif d'irradiation selon l'une des revendications 5 à 11, **caractérisé en ce qu'**une tête d'irradiation (15) contenant la source de rayonnement (3) et le collimateur (1) est disposée sur un bras de robot qui est réalisé de sorte qu'il peut amener la tête d'irradiation (15) dans n'importe quelle position spatiale et orientation angulaire spatiales.

14. Dispositif d'irradiation selon l'une des revendications 5 à 13, **caractérisé en ce que** le collimateur (1) est logé coulissant de part et d'autre sur un rail sphérique pour des mouvements de pivotement dans une zone annulaire spatiale limitée, l'axe (11) étant orienté toujours en direction de la source d'irradiation (3).

15. Dispositif d'irradiation selon l'une des revendications 5 à 14, **caractérisé en ce que** chaque diaphragme à iris (5, 6) dispose d'un entraînement (19).

16. Dispositif d'irradiation selon la revendication 15, **caractérisé en ce que** la commande pilote l'ouverture respective de diaphragmes (8, 8', 8") en fonction de la divergence des rayons (2').

17. Dispositif d'irradiation selon l'une des revendications 5 à 14, **caractérisé en ce que** les diaphragmes à iris (5, 6) sont actionnables au moyen d'un entraînement unique (19).

18. Dispositif d'irradiation selon la revendication 17, **caractérisé en ce qu'**un mécanisme de réglage provoque l'ouverture respective de diaphragme (8. 8', 8'') en fonction de la divergence des rayons (2').

19. Dispositif d'irradiation selon l'une des revendications 5 à 18, **caractérisé en ce que** les lamelles de diaphragmes (9, 9', 9'', 9"', 9"", 9""') de chaque diaphragme à iris (5, 6) sont guidées dans au moins une plaque de guidage (21) contenant une partie de guidage (18') des guides linéaires (18).

20. Dispositif d'irradiation selon la revendication 18, **caractérisé en ce qu'**une came disque (22) est reliée activement avec les lamelles de diaphragmes (9, 9', 9", 9"', 9'''', 9''''') et **en ce que** l'entraînement (19) provoque les mouvements de réglage (13) des lamelles de diaphragmes (9, 9', 9", 9"', 9"", 9""') par un mouvement de rotation relatif entre la plaque de guidage (21) et la came disque (22).

21. Dispositif d'irradiation selon la revendication 18 ou 19, en ce que les lamelles de diaphragmes (9, 9', 9'', 9"', 9'''', 9''''') de chaque diaphragme à iris (5, 6) sont guidées également le long de la face opposée au moyen d'une autre plaque de guidage (21').

22. Dispositif d'irradiation selon la revendication 16 à 20, **caractérisé en ce que** l'entraînement (19) s'engage entre deux diaphragmes à iris (5, 6) pour actionner de là les deux diaphragmes à iris (5, 6).

23. Dispositif d'irradiation selon la revendication 16 à 21, **caractérisé en ce que** l'entraînement (19) est associé à un diaphragme à iris (5) et l'entraînement de l'autre diaphragme à iris (6) est provoqué par des entraîneurs de l'entrainement, les éléments de transmission de force étant réalisés pour provoquer des ouvertures de diaphragme (8, 8', 8'') correspondant à la divergence des rayons (2').

24. Dispositif d'irradiation selon la revendication 16 à 22, **caractérisé en ce que** les moyens de réglage (7) sont réalisés pour plusieurs introductions de force de l'entraînement (19).

25. Dispositif d'irradiation selon la revendication 24, **caractérisé en ce que** qu'au moins une introduction de force est associée à un engrenage (20) qui provoque un rapport des ouvertures de diaphragme (8, 8', 8'') correspondant à la divergence des rayons (2').

26. Dispositif d'irradiation selon la revendication 25, **caractérisé en ce qu'**au moins un engrenage (20) présente au moins un organe de transmission échangeable ou réglable pour régler le collimateur (1) sur différentes divergences de rayon.

27. Dispositif d'irradiation selon l'une des revendications 20 à 26, **caractérisé en ce que** les cames disque (22) présentent des cames d'entraînement (23) disposées en étoile qui agissent sur des entraîneurs (24) en forme de boulon des lamelles de diaphragmes (9, 9', 9'', 9''', 9'''', 9''''').

28. Dispositif d'irradiation selon la revendication 27, **caractérisé en ce que** les entraîneurs (24) en forme de boulon sont des galets.

29. Dispositif d'irradiation selon l'une des revendications 5 à 28, **caractérisé en ce qu'**il est prévu deux diaphragmes à iris (5, 6) décalés de 30° dans le sens de rotation et comportant six lamelles.
